(19) Europäisches Patentamt
European Patent Office
Office européen des brevets

(11) Publication number: **0 238 982 B1**

(12) # EUROPEAN PATENT SPECIFICATION

(45) Date of publication of patent specification: **10.03.93** (51) Int. Cl.[5]: **C07D 451/10**, A61K 31/46

(21) Application number: **87103868.3**

(22) Date of filing: **17.03.87**

(54) **Anticholinergic compounds, pharmaceutical compositions and method of treatment.**

(30) Priority: **17.03.86 US 839941**

(43) Date of publication of application:
**30.09.87 Bulletin 87/40**

(45) Publication of the grant of the patent:
**10.03.93 Bulletin 93/10**

(84) Designated Contracting States:
**AT BE CH DE ES FR GB GR IT LI LU NL SE**

(56) References cited:
**FR-M- 6 937**
**US-A- 2 814 623**
**US-A- 3 649 630**
**US-A- 4 160 099**

**J.Med.Chem. 1980, 23, 474**

**Adv.Drug Res.13, 255 (1984)**

(73) Proprietor: **UNIVERSITY OF FLORIDA**
**207 Tigert Hall**
**Gainesville, Florida 32611(US)**

(72) Inventor: **Hammer, Richard H.**
**1704 N. W. 68th Terrace**
**Gainesville Florida 32605(US)**
Inventor: **Bodor, Nicholas S.**
**7211 SW 97th Lane**
**Gainesville Florida 32608(US)**

(74) Representative: **Redies, Bernd, Dr. rer. nat.**
**COHAUSZ & FLORACK Patentanwaltsbüro**
**Schumannstrasse 97 Postfach 14 01 20**
**W-4000 Düsseldorf 1 (DE)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.5x/3.0.1)

## Description

Thepresent inventionrelates to certain novel anticholinergic and mydriatic compounds as well as pharmaceutical compositions containing the novel compounds and methods of treatment of animals and humans in need thereof.

Prior Art

Commercially available anticholinergic drugs such as atropine andscopolamine and their synthetic analogs all share a variety of undesirable side effects. In the elderly, exciterment, agitation and drowsiness are frequently observed even in small doses. Dangerous central nervous system mediated psychotic reactions and behavioral disturbances have occurred in children after topical ocular administration. Ophthalmic use may also induce local side effects such as transient stinging, allergic lid reactions, follicular conjunctivitis, edema and photophobia. See Toxicology of Commercial Products, R.E. Gosselin et al, Eds. (Williams & Wilkins, Balt., 4th Ed., 1976) Sec. III, pp. 43-46.

Mydriatic agents are an important class of compounds that are used to dilate the pupil. Mydriasis is required during ophthalmicexaminations, in order to provide for a more complete examinations, in order to provide for a more complete examination of the fundus, the vitreous and the periphery of the lens, and in various surgical procedures such as those reported by Freeman et al., American Intra-Ocular Society Journal7:172-173 (1981) (e.g., vitrectomy, lens extraction, andintraocular lens implantation). Commercially available mydriatic drugs such as atropine, scopolamine, homatropine and their synthetic analogs all suffer from several disadvantages. Because the mydriasis induced by these agents causes blurred vision and is of a relatively long duration, i.e., several hours, it is necessary to virtually immobilize the patient after the ophthalmic wxamination until the mydriasis subsides and the patient can resume normal activieties. Ophthalmic use of these agents may also induce local side effects such as transient stinging, allergic lid photophobia. See R.E. Gosselin et al supra.

USP 2814623 describes certain esters of N-methylscopolamine quaternary ammonium salt having high anticholinergic activity but lack appreciable CNS activity. USP 3649630 describes homatropine dipropylacetate which is said to have good antiulcer activity but very little mydriatic and antisecretory activity. F.R.-M 6937 describes derivatives of norscopolamine and the corresponding acid addition and quaternary salt having anticholinergic properties. USP 4160099 describes labile quaternary ammonium salts/esters of atropine as transient derivatives. Bodor in J.Med.Chem. 1980, 23, 474 and in Adv. Drug Res. 13, 255 (1984) describes the same specific group of anticholinergic agents which are "soft" in nature but which are quite different structurally from the compounds described hereinafter.

It is an object of the present invention to provide novel anticholinergic compounds which exhibit anticholinergic properties and which induce mydriasis as well as pharmaceutical compositions and methods of treatment embodying those compounds which are more effective and less toxic than those presently available.

SUMMARY OF THE INVENTION

The foregoing and other objects are realized by the present invention which provides certain novel compounds having the formula:

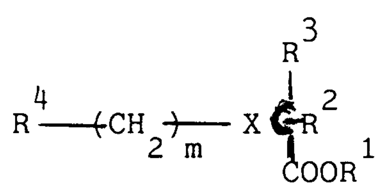

(I)

wherein

$R^1$ is a straight or branched chain alkyl or cycloalkyl group having up to 8 carbon atoms;

$R^2$ is an aryl, cycloalkyl or alkyl group having up to 8 carbon atoms;

$R^3$ is H or a group defined by

$R_2$;

X is

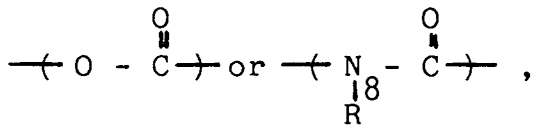

wherein $R^8$ is H or a straight or branched chain alkyl group having up to 5 carbon atoms;
$m_4$ is an integer from 0 to 4;
$R^4$ is

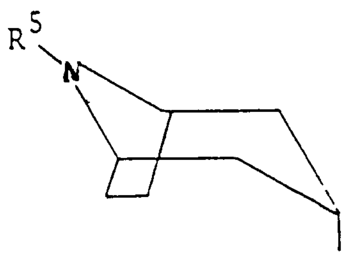

or

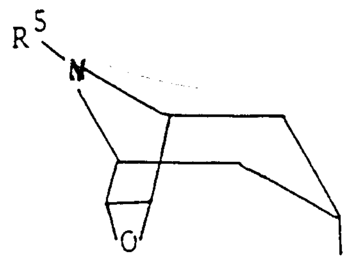

,

wherein $R^5$ is an alkyl group having up to 5 carbon atoms;
or $R^4$ is

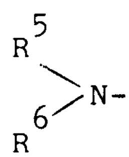

wherein $R^5$ and $R^6$ may be the same or different and are alkyl groups having up to 5 carbon atoms;
or $R^4$ is an ortho-, meta- or para-substituted alkyl pyridine of the formula

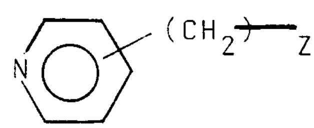

wherein Z is an integer from 0 to 3;
or a quaternary ammonium salt of a compound of formula (I) with a compound of the formula $R^7Y$ wherein $R^7$ is an alkyl group having up to 5 carbon atoms and Y is a pharmaceutically acceptable anion.

The compounds of formula (I) exhibit anticholinergic and/or mydriatic properties.

The invention further provides pharmaceutical compositions in unit dosage form comprising an anticholinergic or mydriasis inducing effective amount of a compound of the above formula and a pharmaceutically acceptable carrier therefor.

## DETAILED DESCRIPTION OF THE INVENTION

The invention is predicated on the discovery that certain esters of inactive polar acidic metabolites of atropine, scopolamine and other compounds, as well as synthetic analogs thereof, are active anticholinergic compounds which are significantly less toxic than their parent free alcohols. Several have also been found to be active mydriatic agents which induce a much shorter duration of mydriasis than the parent free alcohols.

For example, the free acids having the structural formula:

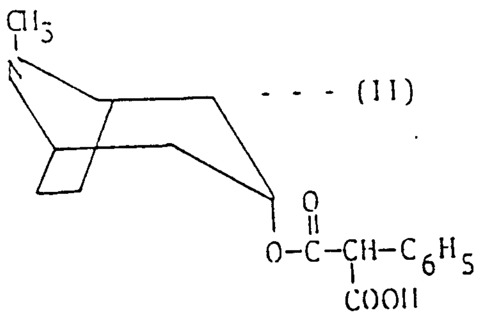
- - - (II)

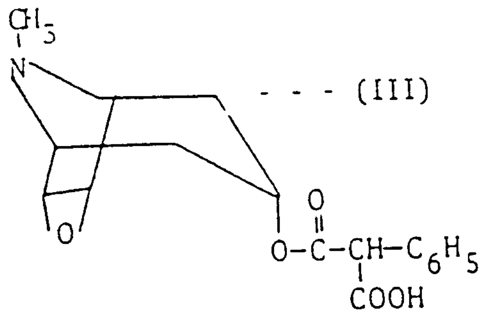
- - - (III)

are anticholinergically inactive metabolites of atropine and scopolamine, respectively. Certain esters of these acids possess anticholinergic and mydriatic properties equivalent to atropine and scopolamine but are far less toxic. The esters are metabolically hydrolyzed to the above inactive acidic metabolites (II) and (III) and non-toxic alcohols when administered to animals or humans.

$R^1$ in the above formula (I) is the esterification residue of a pharmaceutically acceptable non-toxic alcohol $R^1OH$ such that the ester group therein, $-COOR^1$ is metabolically hydrolyzable to the free alcohol and the inactive acidic metabolite -COOH. $R^1$is most preferably lower alkyl, e.g., methyl, ethyl, propyl, etc.

The quaternary ammonium derivatives are the preferred anticholinergic and mydriatic compounds in that they possess higher anticholinergic activities than the non-derivatized compounds. The quaternary substituents may be any non-toxic lower alkyl group, most preferably methyl, and Y may be any pharmaceutically acceptable anion, preferably halogen, sulfate, alkylsulfate or alkylsulfonate.

For purposes of illustration, the compounds of the present invention are referred to as "soft drug" analogs or derivatives of the corresponding active alcohols.

Soft drug mydriatic agents according to the present invention are illustrated in the following Table A which exemplifies several drugs based upon the above structural formula I.

The esters are prepared in two steps to the tertiary compounds and in three steps to the quaternary compounds from the phenylmalonic acid.

The following reaction scheme is illustrative of the method:

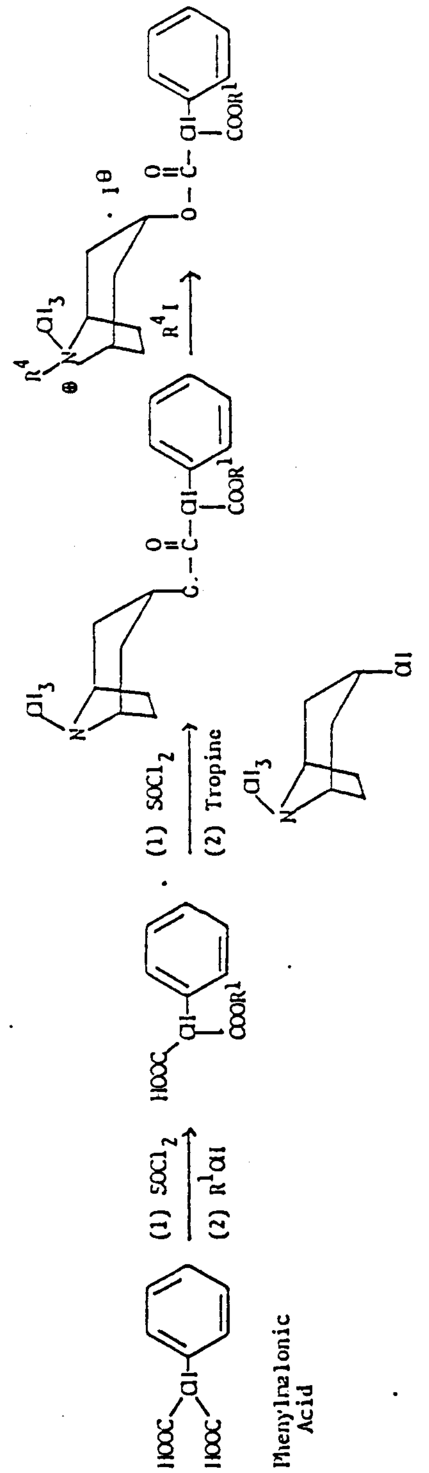

Phenylmalonic Acid

## TABLE A

1. Tropicamide Soft Drug

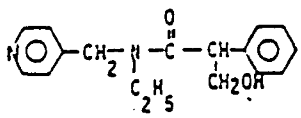

Tropicamide

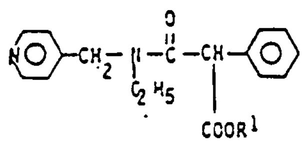

Soft Drug

$R^1$=lower alkyl (1 to 8 carbon atoms)

$R^2$=Phenyl or other aryl or cycloakyl

$R^3$=H

X=-N(R8)-C(=O)-(amide)  $R^8$= $-C_2H_5$

m=0

$R^4$= 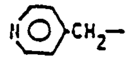  z=1

2. Ipratropium Soft Drug

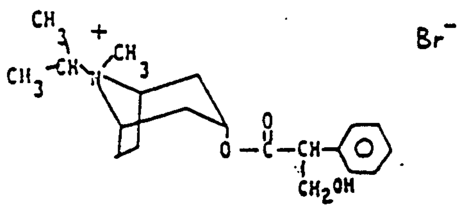

Ipratropium Bromide

$R^1$=lower alkyl (1 to 8 carbon atoms)

$R^2$=Phenyl

$R^3$=H

X=-O-C(=O)-(ester)

m=0

TABLE A (cont.)

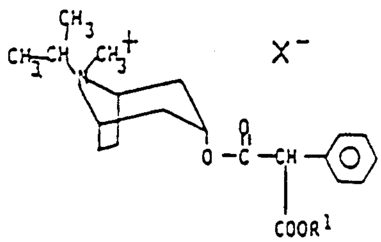

Soft Drug

$R^4$ = 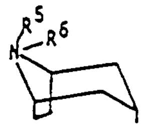

$R^5$= $(CH_3)_2CH-$

$R^6$=$-CH_3$

$X^-$=anion, preferably halogen, alkylsulfate, sulfate, alkylsulfonate

3 Cyclooentolate Soft Drug

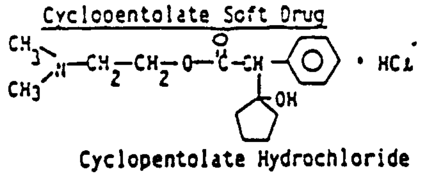

Cyclopentolate Hydrochloride

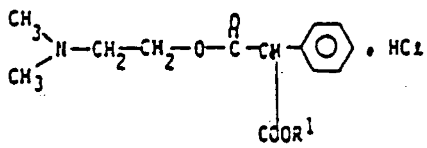

Soft Drug

$R^1$=lower alkyl (1 to 8 carbon atoms)

$R^2$=Phenyl or other aryl or cycloalkyl

$R^3$=H

$X=-O-\overset{O}{\overset{\|}{C}}-$(ester)

m = 2

$R^4$= 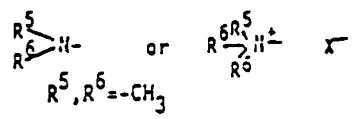

$R^5,R^6=-CH_3$

where $R^5$, $R^6$, $R^7$ = lower alkyl (1 to 5 carbon atoms), preferably methyl and $X^-$ = anion, preferably halogen, alkysulfate, sulfate, alkylsulfonate.

Soft drug anticholinergic agents according to the present invention are illustrated in the following Table B which exemplifies several drugs based upon above structural formula I.

TABLE B

Following the general procedure and substituting the appropriate reactants affords the following novel soft anticholinergic compounds:

| $R^1$ | $R^2$ | $R^3$ | $R^4$ | n (0 to 4) | X | $X^-$ |
|---|---|---|---|---|---|---|
| Lower alkyl, cyclic, straight or branched chain alcohols (1 to 8 carbon atoms) | 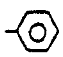 | H | 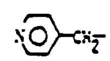 | 0 | 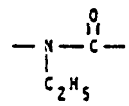 | Any pharmaceutically acceptable anion, halogen, sulfate, alkylsulfate or alkylsulfonate |
| $CH_3CH_2$- | 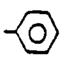 | H | 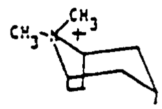 | 0 | 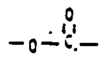 | $I^-$ |
| $CH_3CH_2CH_2$- | 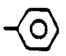 | H | 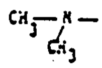 | 2 | 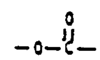 | -- |
| 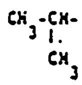 | 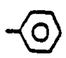 | 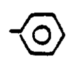 | 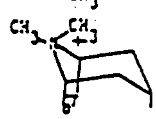 | 0 | 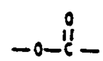 | $CH_3SO_4^-$ |
| $CH_3(CH_2)_2CH_2$- | 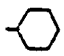 | 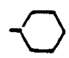 | 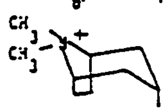 | 0 | 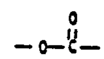 | $CH_3SO_3^-$ |

The rates of metabolic hydrolysis of the active esters may be controlled by selection of the esterifying alcohol.

The invention is illustrated by the following non-limiting examples.

EXAMPLE 1

Methyl hydrogen phenylmalonate

A mixture of phenylmalonic acid (3.6 g, 0.02 mol) boron trifluoride etherate (2.4 ml, 0.02 mol) and 0.8 ml of anhydrous methanol (0.02 mol) was refluxed and stirred for 24 hours. After cooling to room temperature, the reaction mixture was filtered and 20 ml of water was added.

The precipitated oil was extracted with $CHCl_3$ (30x3). The combined extracts were washed with water and dried over anhydrous $MgSO_4$ and evaporated under reduced pressure. The obtained oily liquid showed nmr spectra is consistent with its structure.

$R_f$ of the acid = 0.52 yield = 57%

$R_f$ of the ester = 0.72.

Preparation of methyl phenylmalonyl chloride

To the obtained oily liquid above was added 5 ml of thionyl chloride. The reaction mixture was heated on an oil bath for one hour under anhydrous condition. The excess thionyl chloride were removed by the addition and the in vacuo distillation of 10 ml of anhydrous benzene. The product was oily and was reacted immediately with tropine base.

Esterification of methyl phenylmalonyl chloride and tropine

Thoroughly dried tropine (1.4 g, 0.01 mol) was added to the freshly prepared methyl hydrogen phenylmalonyl chloride. The mixture was heated for 5 hrs at 100°, while stirring, under anhydrous condition. The mixture turned to brown and gas evolved. After cooling to room temperature and filtration the mixture was treated with 10 ml of water. The clear solution was adjusted to pH 9 with saturated $Na_2CO_3$ solution, extracted with ethyl ether and dried over anhydrous $MgSO_4$. The ethereal solution was filtered and distilled under reduced pressure to produce an oily product.

EXAMPLE 2

Preparation of Ethyl (±)-α-(carboxy)-8-methyl-8-azabicyclo[3•2•1]oct-3-yl-benzeneacetate

a) Preparation of ethyl hydrogen phenylmalonate (1):

Phenylmalonic acid (13.5 g, 0.07 mol) in dry ether (40 ml) was treated with thionyl chloride (8.92 g, 5.4 ml, 0.07 mol) and one drop of N,N-dimethyl formamide. The mixture was heated at 40-50 °C. for 3 hours. The clear solution was evaporated under reduced pressure to remove any residual thionyl chloride. The oily residue redissolved in dry ether (40 ml), the solution was treated with ethyl alcohol (0.075 mol, 4.1 cc) and refluxed for 2 hours. The reaction mixture was cooled to room temperature and washed with water. The organic layer was extracted with saturated solution of sodium bicarbonate until alkaline. The combined extracts were washed with ether and the aqueous layer acidified with 5N HCl to pH1. The precipitated oil was extracted with $CH_2Cl_2$ (3x50). The combined extracts were washed with water (4x50) and dried over anhydrous $MgSO_4$. The oil product crystallized on standing.

Yield: - 7.2 g (49.4%)

M.p.: 78-9 °C (as reported).

b) Preparation of ethyl phenylmalonyl chloride

Ethyl hydrogen phenylmalonate (2.08 g, 0.01 mol) mixed with thionyl chloride (5 ml) and heated at 70 °C for one hour under anhydrous condition. The obtained liquid was evaporated under reduced pressure. Anhydrous benzene (10 ml) was added to the residue and redistilled again to remove any residual thionyl chloride.

c) Esterification of ethyl phenylmalonyl chloride and tropine

The previously obtained acid chloride (0.01 mol) dissolved in 10 ml of dry benzene was added dropwise with stirring for half an hour to a solution of dry tropine (2.8g, 0.02 mol) in dry benzene. The mixture was stirred at room temperature for 24 hours and filtered. The filtrate was washed with water until neutral. The organic layer was dried over anhydrous $MgSO_4$ and the solvent evaporated. The obtained oily base was converted into oxalate salt by adding an ether solution of oxalic acid to a solution of the base in ether. By scratching the mixture a white solid product was obtained. When cooled in a deep freezer, then washed with cold ether and dried for 24 hours in a desiccator over $CaCl_2$, the crystalline product represented 78% conversion to oxalate.

Recrystallization was carried out from methanol-ether mixture.

$R_f$ of the free base = 0.4

Yield of the free base = 75%

Yield of the oxalate = 78%

M.p. 135-7 °C.

| Anal for $C_{19}H_{25}O_4N.(COOH)_2$ | | | |
|---|---|---|---|
| | C% | H% | N% |
| Theoretical | 59.85 | 6.45 | 3.32 |
| Found | 59.88 | 6.52 | 3.30 |

$^1$HNMR ($CDCl_3$) = δ 7.31 (s, 5H, $C_6H_6$) 5.13-4.88 (s, 1H, $C_3$ tropine) 4.53 (s, 1H,

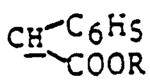

),

4.19 (q, 2H, J = 7Hz, $-COOCH_2CH_3$) 3.29-2.9 (br.s, 1H, $C_1$ + $C_5$ of tropine), 2.83-1.6[m, 11H, $N-CH_3$ (s, 2.25) + , bicyclic envelop of tropine.

According to the microanalytical data and NMR spectra, the obtained compound has the following structure:

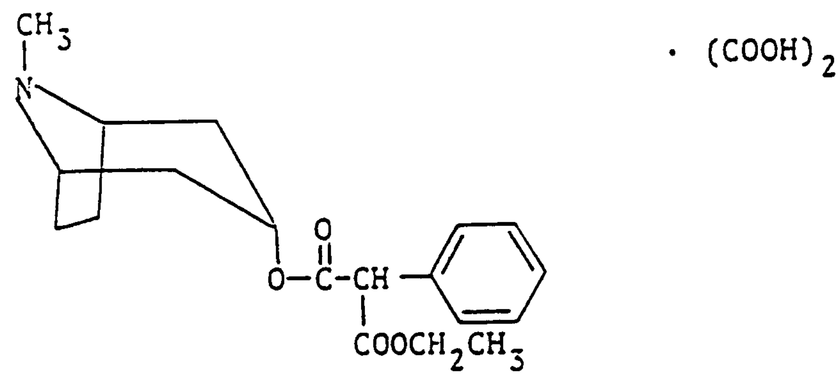

EXAMPLE 3

Synthesis of Methyl (±)-α-(carboxy)-8-methyl-8-azabicyclo[3•2•1]oct-3-yl-benzeneacetate

a) Synthesis of methyl hydrogen phenylmalonate (new method)

Phenylmalonic acid (13.5 g, 0.07 mol) in dry ether (40 ml) was treated with thionyl chloride (8.92 g, 5.4 ml, 0.071 mol) and one drop of N,N-dimethyl formamide. The mixture was heated at 40-50 C for 3 hours. The clear solution was evaporated under reduced pressure to remove any residual thionyl chloride. The oily residue was redissolved in dry ether (40 ml) and the solution was treated with methyl alcohol (0.075 mol, 3 ml) and refluxed for 2 hours. The reaction mixture was cooled to room temperature and washed with water. The organic layer was extracted with saturated solution of sodium bicarbonate until alkaline. The combined extracts were washed with ether and the aqueous layer acidified with 5N HCl to pH1. The precipitated oil was extracted with $CH_2Cl_2$ (3x50). The combined extracts were washed with water (4x50) and dried over anhydrous $MgSO_4$. The oil product crystallized on standing.

Yield - 6 g (41.2%)

M.p. - 86-88 ° C as reported.

b) Synthesis of methyl phenylmalonyl chloride

The previously prepared ester (2.94 g, 0.01 mol) was mixed with thionyl chloride (5 ml) and heated at 70 ° C for one hour under anhydrous condition. The obtained liquid was evaporated under reduced pressure. Anhydrous benzene (10 ml) was added to the residue and redistilled again to remove any residual thionyl chloride.

c) Esterification of methyl phenylmalonyl chloride and tropine

The previously prepared acid chloride was dissolved in 10 ml of dry benzene and was added dropwise with stirring for half an hour to a solution of dry tropine (2.8 g, 0.02 mol) in dry benzene. The mixture was stirred at room temperature for 24 hours and filtered. The filtrate was washed with water until neutral. The organic layer was dried over anhydrous $MgSO_4$ and the solvent evaporated. The obtained oily base was purified by conversion into oxalate salt by adding an ether solution of oxalic acid to a solution of the base in ether. By scratching the mixture and keeping in a refrigerator overnight, a white solid product was separated. Rapid filtration and drying in a vacuum desiccator over anhydrous $CaCl_2$ was carried out.

Recrystallization from methanol-ether mixture gave white crystalline product.

$R_f$ of the free base = 0.41

Yield of the free base = 1 g (64.5%)

Yield of the oxalate = 1.5 g (35.7%)

M.p. - 89.91 ° C.

| Anal. for $C_{18}H_{23}O_4N.(COOH)_2.H_2O$: | | | |
|---|---|---|---|
| | C% | H% | N% |
| Theoretical | 56.47 | 6.39 | 3.29 |
| Found | 56.48 | 6.2 | 2.94 |

$^{1}$HNMR ($CDCl_3$): δ 7.33 (s, 5H, $C_6H_5$), 5.2-4.8 (br. s, 1H, $C_3$ tropine), 4.6 (s, 1H,

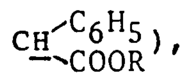

3.4 (s, 3H -$OCH_3$), 3.29-2.9 (s, 2H, $C_1$ + $C_5$ of tropine), 2.23-1.4 [(m, 11H, bicyclic envelop + $NCH_3$ (s, 223)].

According to the microanalytical data and NMR spectra, the compound has the following structure:

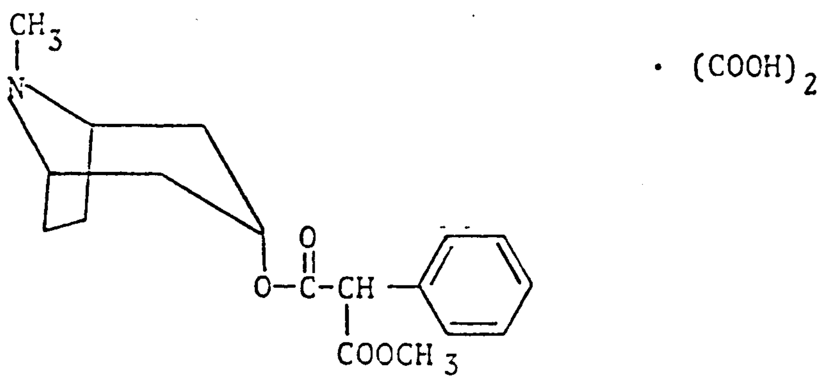

EXAMPLE 4

Synthesis of isopropyl (±)-α-(carboxy)-8-methyl-8-azabicyclo[3•2•1]oct-3-yl-benzeneacetate

a) Synthesis of isopropyl hydrogen phenylmalonate

Phenylmalonic acid (13.5 g, 0.07 mol) in dry ether (40 ml) was treated with thionyl chloride (8.92 g, 5.4 ml, 0.07 mol) and one drop of N,N-dimethyl formamide. The mixture was heated at 40-50 °C for 3 hours. The clear solution was evaporated under reduced pressure to remove any residual of thionyl chloride. The oily residue was redissolved in dry ether (40 ml), and the solution treated with isopropyl alcohol (0.075 mol, 5.76 ml), refluxed for 2 hours and worked up as before to give the titled compounds.

Yield = 60% (as reported)

M.p. - 64-66 °C (as reported).

b) Synthesis of isopropyl phenylmalonyl chloride

The previously prepared monoester (2.22 g, 0.01 mol) was mixed with thionyl chloride (5 ml) and heated at 70 °C for one hour under anhydrous condition. The obtained liquid was evaporated under reduced pressure. Anhydrous benzene (10 ml) was added and the mixture redistilled again.

c) Esterification of isopropyl phenylmalonyl chloride and tropine

The previously prepared acid chloride was dissolved in 10 ml of dry benzene and added dropwise with stirring for half an hour to a solution of dry tropine (2.8 g, 0.02 mol) in dry benzene. The mixture was stirred at room tenperature for 24 hours and filtered. The filtrate was washed with water until neutral. The organic layer was dried over anhydrous $MgSO_4$ and the solvent evaporated. The obtained oily base was purified by

formation of oxalate salt. An ether solution of oxalic acid was added to an ether solution of the base. By scratching the mixture and keeping in a deep freezer for 24 hours, a white solid product was obtained. Filtration followed by drying in a vacuum desiccator over $CaCl_2$ was carried out.

Recrystallization from methanol-ether mixture gave a white crystalline product.

$R_f$ of the base - 0.3

Yield of the base - 2.5 g (72.4%)

Yield of the oxalate - 3 g (68.9%)

M.p. - 78-80 °C.

| Anal. for $C_{20}H_{27}O_4N.(COOH)_2$. | | | |
|---|---|---|---|
| | C% | H% | N% |
| Theoretical | 60.68 | 6.71 | 3.21 |
| Found | 60.41 | 6.77 | 3.13 |

$^1$HNMR ($CDCl_3$): δ 7.23 (s, 5H, $C_6\underline{H}_5$), 5.2-4.8 (br. s, 1H, $C_3$ tropine), 4.46 (s, 1H,

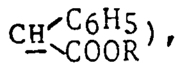

3.1-2.91 (br. s, 2H, $C_1$ + $C_5$ of tropine), 2.3-1.44 [(m, 11H, bicyclic envelope), + 2.2 (s, 3H, -N-$C\underline{H}_3$), 1.23 (d, 6H,

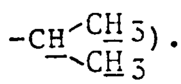

According to both microanalytical data and NMR spectra, the obtained compound has the following structure:

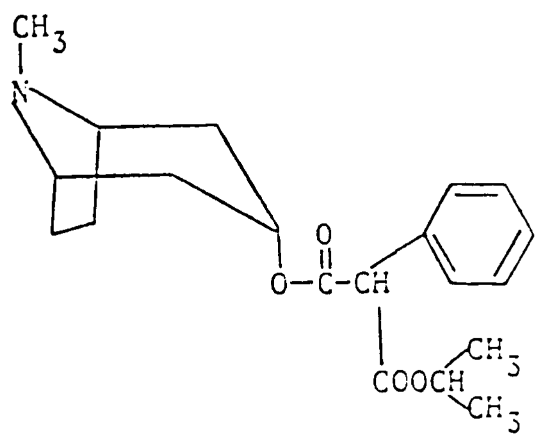

· $(COOH)_2$

EXAMPLE 5

Synthesis of cyclohexyl (±)-α-(carboxy)-8-methyl-8-azabicyclo-[3.2.1]oct-3-yl-benzeneacetate

a) Preparation of cyclohexyl hydrogen phenylmalonate

Phenylmalonic acid (13.5 g., 0.07 mol) in dry ether (40 ml) was treated with thionyl chloride (8.92 g, 5.4 ml, 0.07 mol) and one drop of N,N-dimethyl formamide. The mixture was heated at 40-50 °C for 3 hours. The clear solution was distilled under vacuum. The oil residue redissolved in dry ether (40 ml), the solution was treated with cyclohexyl alcohol (0.075 mol, 7.9 cc) and refluxed for 2 hours. The mixture was worked

up as before to give an oily product which crystallized on standing. Recrystallization was carried out from benzene-petroleum ether (40-60 ° C) mixture to give a white crystalline product.

$R_f$ - 0.76

M.p. - 80-2 ° C

Yield - 12.5 g (64.14%).

| Anal. for $C_{15}H_{18}O_4$ | | |
|---|---|---|
| | C% | H% |
| Theoretical | 68.69 | 6.9 |
| Found | 68.79 | 6.92 |

$^1$HNMR ($CDCl_3$): $\delta$ 8.96 (s, 1H, -COOH), 7.3 (s, 5H, $C_6H_5$), 4.6 (s, 1H,

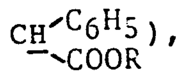

1.96-1.10 (br. s, 11H, $C_6H_{11}$).

According to the previous data, the synthesized compound has the following structure:

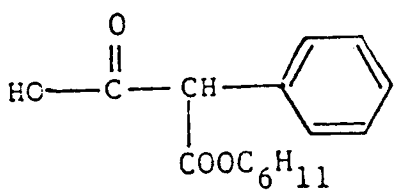

b) Synthesis of cyclohexyl phenylmalonyl chloride

The cyclohexyl ester prepared above (2.6 g, 0.01 mol) was mixed with thionyl chloride (5 ml) and heated at 70 ° C for one hour under anhydrous condition. The obtained liquid was evaporated under reduced pressure. Anhydrous benzene (10 ml) was added to the residue and redistilled again to remove any residual thionyl chloride. The oily product was solidified on standing and was used for the next step without further purification.

c) Esterification of cyclohexyl phenylmalonyl chloride and tropine

The acid chloride was dissolved in 10 ml of dry benzene and added dropwise with stirring for half an hour to a solution of dry tropine (2.8 g, 0.02 mol) in dry benzene. The mixture was stirred at room temperature for 24 hours and filtered. The filtrate was washed with water until neutral. The organic layer was dried over anhydrous $MgSO_4$ and the solvent evaporated. The obtained oily base was converted into oxalate salt by adding an ether solution of oxalic acid to a solution of the base in ether. By scratching the mixture and cooling in a refrigerator, a white solid product was separated. Filtration and drying over $CaCl_2$ in a vacuum desiccator was carried out. On recrystallization from methanol-ether mixture, a white crystalline product was obtained.

$R_f$ - 0.22

Yield of the base - 3 g 77%

Yield of the oxalate - 3.1 g 63%

M.p - 160 ° -2 ° C.

| Anal. for $C_{23}H_{31}NO_4.(COOH)_2$ | | | |
|---|---|---|---|
| | C% | H% | N% |
| Theoretical | 63.15 | 6.99 | 2.94 |
| Found | 63.05 | 7.26 | 2.78 |

$^1$HNMR ($CDCl_3$): $\delta$ 7.33 (s, 5H, $C_6\underline{H}_5$), 4.61(s, 1H,

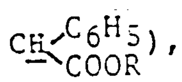

3.2-2.9 (br s, 2H, $C_1$ + $C_5$ of tropine); 2.23-1.06[m, 21H, bicyclic envelop + N-$CH_3$ (s, 2.23) + $C_6H_{11}$ (br s, 1.66-1.06)].

According to the microanalytical data and NMR spectra the obtained compound has the following structure:

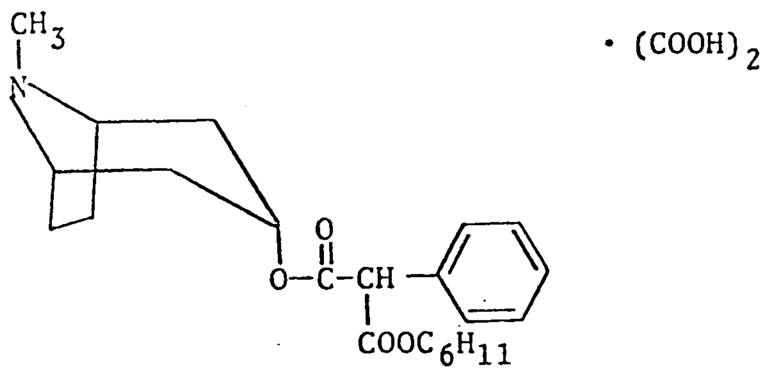

EXAMPLE 6

Preparation of: Tropanyl hydrogen phenylmalonate (±)-α-(carboxy)-8-methyl-8-azabicyclo-[3•2•1]oct-3-yl benzeneacetic acid

Phenylmalonic acid (2.5 g, 0.014 mol) in dry ether (10 ml) was treated with thionyl chloride (1.78 g, 1.09 ml, 0.01 mol), and N,N-dimethyl formamide (two drops). The mixture was heated at 50°-60°C for 2 hr. Ether and excess thionyl chloride were removed and three separate portions of dry benzene were added and removed the same way. The residue (1.9 gm, 0.01 mol) was dissolved in 20 ml dry benzene and added dropwise over a period of 0.5 hr to a stirred solution of dry tropine (2.82 g, 0.02 mol) in 10 ml dry benzene. The mixture was stirred at ambient temperature for 24 hr and filtered. The filtrate was washed with water until neutral and the organic layer was dried over anhydrous $MgSO_4$ and evaporated. The oily product was transformed to the corresponding oxalate by addition of an ethereal solution of oxalic acid to the solution of the compound in ether. A white solid product was obtained by scratching the ethereal solution and keeping in a refrigerator overnight.

The white solid was isolated by filtration and thoroughly washed with ether and dried to give 0.7 gm (23%). $R_f$ ($CHCl_3$: MeOH 3:1) = 0.23, m.p. 112-115°C.

| Anal. for $C_{17}H_{21}O_4N\ (COOH)_2\ 0.5H_2O$ | | | |
|---|---|---|---|
| | C% | H% | N% |
| Theoretical | 56.71 | 6.01 | 3.48 |
| Found | 56.94 | 6.26 | 3.32 |

$^1$HNMR ($CDCl_3$) $\delta$ 7.16 (s, 5H, $C_6\underline{H}_5$); 4.88 (s, 1H,

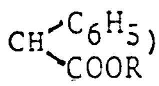 );

3.13-2.86 (br s, 2H, $C_1 + C_5$ of tropine); 2.23-1.7 [m, 11H, bicyclic envelope of tropine + N-$CH_3$ (s, 2.23)].

According to the above data, the obtained compound has the following structure:

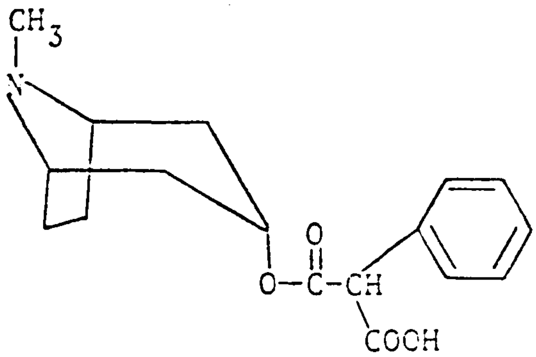

· $(COOH)_2$

EXAMPLE 7

Preparation of: Cyclohexyl (±)-α-(carboxy)-8-methyl-8-azabicyclo-[3•2•1]oct-3-yl benzeneacetate methiodide

A solution of cyclohexyl (±)-α-carboxymethyl-8-methyl-8-azabicyclo (3•2•1)oct-3-yl benzeneacetate (3.85 g, 0.01 mol) and 10 ml of methyl iodide in 30 ml of dry benzene was stirred at room temperature for 6 hr. The mixture was filtered and the solid was dried and recrystallized from methanol-ether mixture to give a yellowish-white solid product.

$R_f$ - 0.35
Yield - (55.9%)
M.p. - 228-230 °C.

| Anal for $C_{24}H_{34}INO_4.0.25\ H_2O$ | | | |
|---|---|---|---|
| | C% | H% | N% |
| Theoretical | 54.19 | 6.53 | 2.63 |
| Found | 54.00 | 6.48 | 2.50 |

$^1$HNMR (DMSO-$d_6$) $\delta$ 7.33 (s, 5H, $C_6\underline{H}_5$); 4.99 (s, 1H,

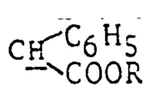 + $C_3$

of tropine); 3.1-1.4 [m, 24H, $N^+CH_3$ (s, 3.1) + $N^+CH_3$ (s, 3) + bicyclic envelop + $C_6H_{11}$ (br s, 1.7-1.4)].

According to the previous data, the obtained compound has the following structure:

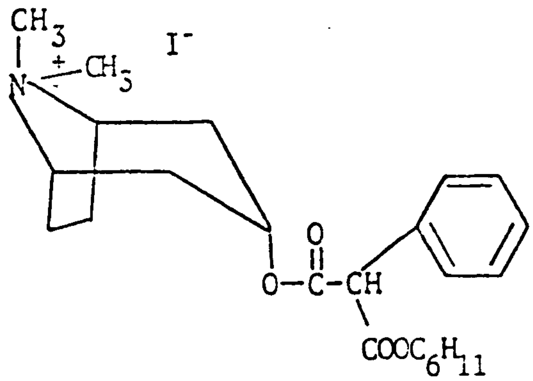

EXAMPLE 8

Preparation of: Ethyl (±)-α-(carboxy)-8-methyl-8-azabicyclo[3•2•1]oct-3-yl benzeneacetate methiodide

A solution of ethyl (±)-α-(carboxy)-8-methyl-8-azabicyclo [3•2•1]oct-3-yl benzeneacetate (3.31 g, 0.01 mol) and 10 ml of methyl iodide in 25 ml of dry benzene was stirred at room temperature for 6 hr. The mixture was filtered and the solid was dried and recrystallized from alcohol-ether mixture.

$R_f$ - 0.41

M.p. - 232-234 (dec)

| Anal. for $C_{20}H_{28}INO_4$ | | | |
|---|---|---|---|
| | C% | H% | N% |
| Theoretical | 50.75 | 5.92 | 2.96 |
| Found | 50.55 | 5.97 | 2.97 |

$^1$HNMR (DMSO-$d_6$) δ 7.28 (s, 5H, $C_6H_5$; 4.96 (s, 1H,

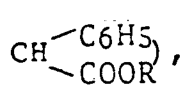

4.13 (q, 2H, J = 7Hz, $-OCH_2CH_3$); 3.23-1.9 [m, 14H, bicyclic envelop + $N^+CH_3$ (s, 3.23) + 1.6 (t, 3H, J = 7Hz, $-OCH_2CH_3$).

According to the previous data the compound has the following structure:

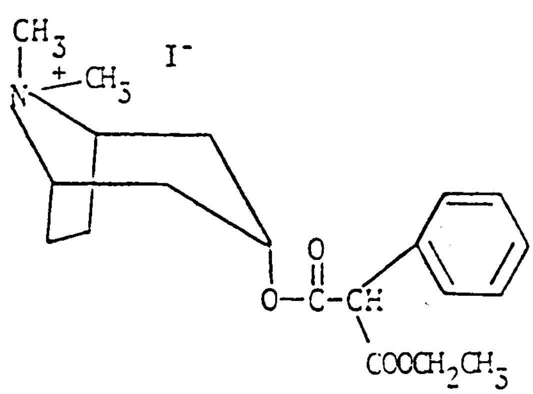

EXAMPLE 9

Preparation of: Methyl (±)-α-(carboxy)-8-methyl-8-azabicyclo[3•2•1]oct-3-yl-benzeneacetate dimethylsulfate

A solution of methyl (±)-α-(carboxy)-8-methyl-8-azabicyclo[3•2•1]oct-3-yl-benzeneacetate in dry ethyl ether and dimethylsulfate (2 x moles of the ester), was stirred at room temperature overnight. The white solid was filtered, dried, and recrystallized from methanol-ether.

M.p. - 138-140 °C

| Anal. for $C_{20}H_{29}NO_8S$•1/2 $H_2O$ | | | | |
|---|---|---|---|---|
| | C% | H% | N% | S% |
| Theoretical | 53.08 | 6.68 | 3.09 | 7.08 |
| Found | 53.06 | 6.65 | 3.01 | 7.14 |

$^1H$ NMR (DMSO-$d_6$) δ 7.4 (s, $C_6H_5$); 5.1 (s,

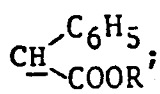

3.8 (s, $-OCH_3$); 3.4 (s, $CH_3SO_4$); 3.1 and 3 (s,

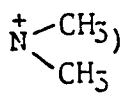

2.7-1.6 (m, bicyclic ring).

According to the previous data the compound has the following structure:

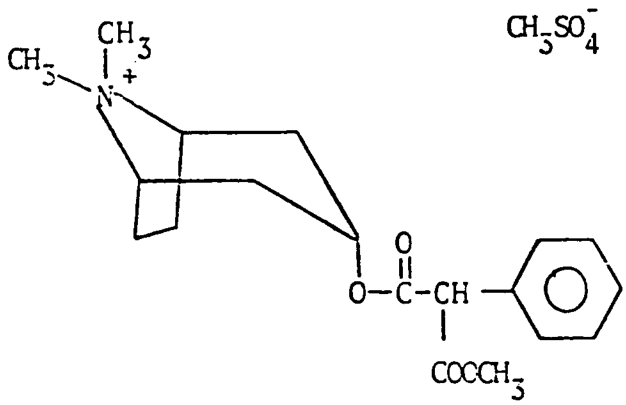

EXAMPLE 10

Preparation of: Ethyl (±)-α-(carboxy)-8-methyl-8-azabicyclo[3•2•1]oct-3-yl-benezeneacetate dimethylsulfate

A solution of ethyl (±)-α-(carboxy)-8-methyl-8-azabicyclo[3•2•1]oct-3-yl-benzeneacetate in dry ethyl ether and dimethylsulfate (2x moles of the ester) was stirred at room temperature overnight. The white solid was filtered, dried and recrystallized from methanol-ether.

M.p. - 165-167 °

| Anal. for $C_{21}H_{31}NO_8S$ | | | | |
|---|---|---|---|---|
| | C% | H% | N% | S% |
| Theoretical | 55.13 | 6.83 | 3.06 | 7.01 |
| Found | 55.10 | 6.87 | 2.96 | 7.02 |

$^1$H NMR DMSO-$d_6$). $\delta$ 7.4 (s, $C_6H_5$); 5.1 (s,

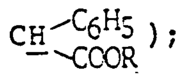

4.2 (q-$\underline{CH_2}CH_3$); 3.4 (s, $CH_3SO_4$); 3.1 and 3 (s,

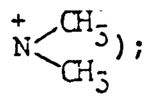

2.8-1.7 (m, bicyclic ring); 1.2 (t, -$CH_2$-$\overline{CH_3}$).

According to the previous data the compound has the following structure:

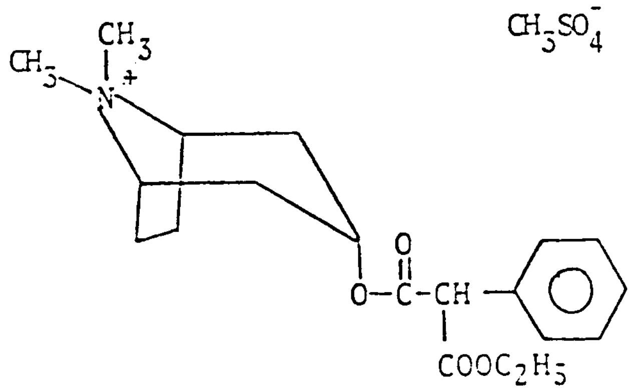

EXAMPLE 11

Preparation of: Isopropyl (±)-α-(carboxy)-8-methyl-8-azabicyclo[3•2•1]oct-3-yl-benzeneacetate dimethylsulfate

A solution of isopropyl (±)-α-(carboxy)-8-methyl-8-azabicyclo[3•2•1]oct-3-yl-benzeneacetate in dry ethyl ether and dimethylsulfate (2x moles of the ester) was stirred at room temperature overnight. The white solid was filtered, dried, and recrystallized from methanol-ether.

M.p. - 128-130°

| Anal. for $C_{22}H_{33}NO_8S$ | | | | |
|---|---|---|---|---|
| | C% | H% | N% | S% |
| Theoretical | 56.03 | 7.05 | 2.97 | 6.80 |
| Found | 55.91 | 7.08 | 2.96 | 6.78 |

$^{1}H$ NMR (DMSO-$d_6$) δ 7.5 (s, $C_6H_5$); 5.1 (s,

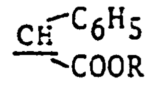

m,

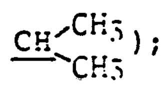

3.4 (s, $CH_3SO_4$); 3.2 and 3.1 (s,

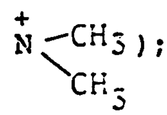

2.8-1.7 (m, bicyclic ring); 1.3 (t,

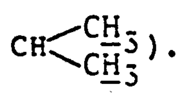

According to the previous data, the compound has the following structure:

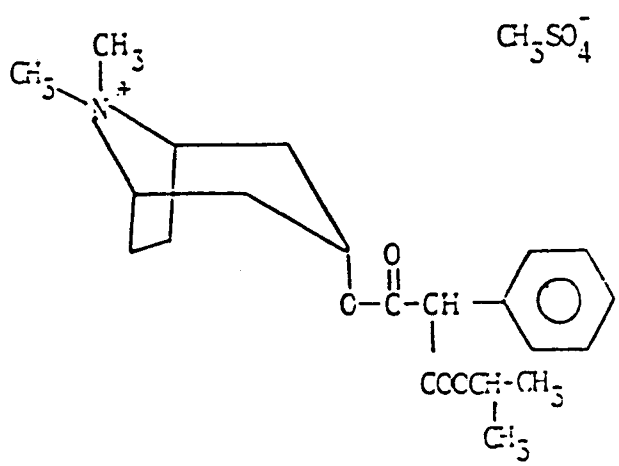

EXAMPLE 12

Preparation of: Cyclohexyl (±)-α-(carboxy)-8-methyl-8-azabicyclo[3•2•1]oct-3-yl-benzeneacetate dimethylsulfate

A solution of cyclohexyl (±)-α-(carboxy)-8-methyl-8-azabicyclo[3•2•1]oct-3-yl-benzeneacetate in dry ethyl ether and dimethylsulfate (2x moles of the ester) was stirred at room temperature overnight. The white solid was filtered, dried, and recrystallized from methanol-ether.

M.p. - 196-198°

| Anal. for $C_{25}H_{37}NO_8S$. 1/2 $H_2O$ | | | | |
|---|---|---|---|---|
| | C% | H% | N% | S% |
| Theoretical<br>Found | 57.67<br>57.91 | 7.36<br>7.15 | 2.69<br>2.68 | 6.16<br>6.17 |

$^1$H NMR (DMSO-$d_6$) δ 7.4 (s, $C_6H_5$); 5.1 (s,

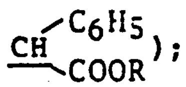

3.8 (s, $CH_3$ $SO_4$); 3.1 and 3 (s,

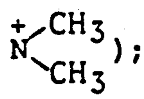

2.8-1.4 (m, bicyclic ring, $C_6H_{11}$).

According to the previous data the compound has the following structure:

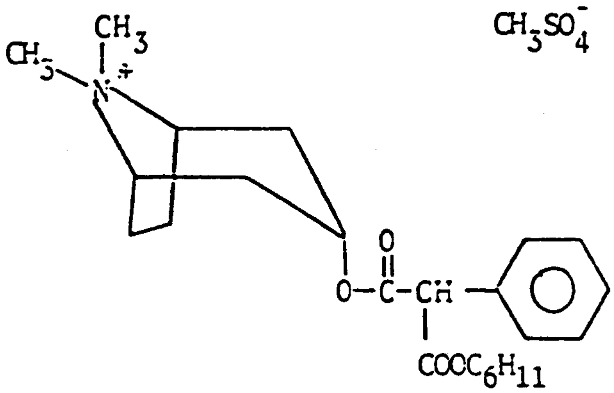

## EXAMPLE 13

### Kinetic Studies of the New Tropanyl Esters

A high pressure liquid chromatography (HPLC) method was used for the determination of rate of hydrolysis of the newly prepared tropanyl esters in aqueous buffered solution at pH 12.0 and in human plasma.

The chromatographic analysis was performed in a system consisting of LCD/Milton Roy Consta Metric III metering pump and LCD UV III Monitor Detector operated at 254 nm. A 30 cm x 3.9 mm (internal diameter) reverse phase ASI/U Bondpak C18 column operated at ambient temperature was used for the analysis. The mobile phase consisted of 32% acetonitrile in 0.01 M potassium dihydrogen phosphate, 0.004 M 1-octanesulfonic acid, sodium in 0.1% acetic acid at a flow rate of 2.3 ml/min.

### Determination of the hydrolytic rate constants in aqueous buffered solution at pH 12 at 37° C

Sodium hydroxide and sodium dibasic phosphate were used to prepare the buffer at pH 12. The ionic strength was maintained at 0.1 M with sodium chloride.

One hundred microliters of a freshly prepared solution of the compound in methanol was added to 10 ml buffered solution, previously equilibrated to 37° C in a water bath and mixed thoroughly to make an

initial concentration of 6 x $10^{-3}$ mol. $liter^{-1}$. Samples of 100 µl were injected into the column at various time intervals. The rate constants, half-lives, and standard errors were calculated and the results are listed in Table

1. The following retention times at a flow rate of 2.3 ml/min using 32% acetonitrile are:

N.B.

Compound (1) had a retention time of 3.0 minutes.
Compound (2) had a retention tire of 7.3 minutes.
Compound (3) had a retention time of 9.3 minutes.
Compound (5) had a retention time of 4.7 minutes.
Compound (7) had a retention time of 3.5 minutes.

At a flow rate of 2.0 ml/min, compound 7 had a retention time of 4.0 min. A mobile phase of 40% acetonitrile was used to separate compounds 4 and 6 at a flow rate of 2.0 ml/min.

Compound (4) had a retention time of 8.0 minutes.
Compound (6) had a retention time of 6.6 minutes.

TABLE 1

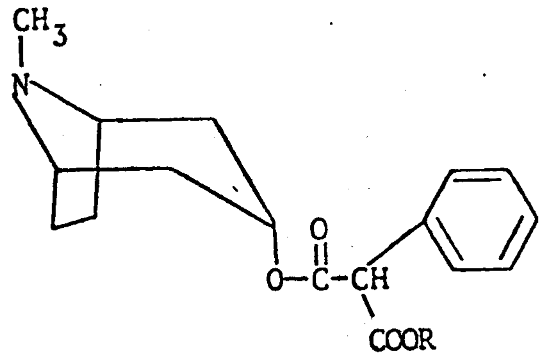

The observed First Order Hydrolytic Rate Constants (k), the standard error (S.E.) and half-life (t 1/2) in 0.01 N sodium hydroxide at pH 12.0, at 37°C

| Compound No. | R | $K^a$ ± S.E ($min^{-1}$) | t 1/2 (min) |
|---|---|---|---|
| 1 | $-CH_3$ | $1.43 \pm 0.12 \times 10^{-1}$ | 4.80 |
| 2 | $-CH_2-CH_3$ | $16.9 \pm 0.15 \times 10^{-2}$ | 4.08 |
| 3 | 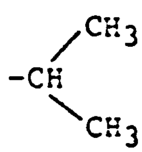 | $30 \pm 0.77 \times 10^{-3}$ | 22.81 |

a) Average of three runs ± S.E.M.

Compound (5)

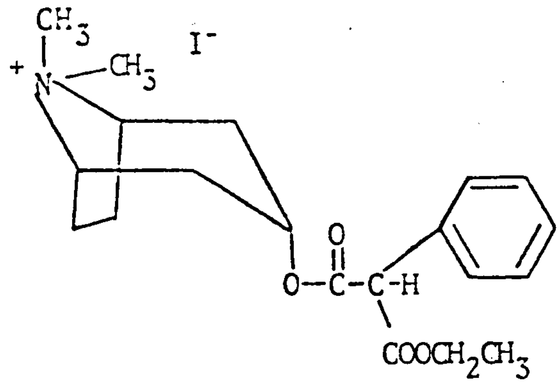

K ± S.E. ($min^{-1}$) = 80.8 ± 0.75 x $10^{-2}$
t 1/2 (min) = 0.85.

The kinetic studies for the hydrolysis of the new tropines in aqueous buffer solution at pH 12.0 and in human plasma was carried out using HPLC method.

EXAMPLE 14

Determination of the hydrolytic rate constants in aqueous buffer at pH 12.0

The previous method adapted for the hydrolysis of compounds 1, 2, 3 and 5 was used for the determination of rate of hydrolysis of compounds 4, 6 and 7. In case of compounds 4 and 6, the mobile phase is 40% acetonitrile, 0.01 M $KH_2PO_4$, 0.04M 1-octanesulfonic acid sodium salt and 0.1% HAC, at a flow rate 2 ml/min. Compounds 4 and 6 had retention times of 8.0 and 6.6 min, respectively, as shown in Table 2. Compound 7 had a retention time of 3.1 min at a flow rate of 2.3 ml/min and 3.5 min at 2.0 ml/min.

TABLE 2

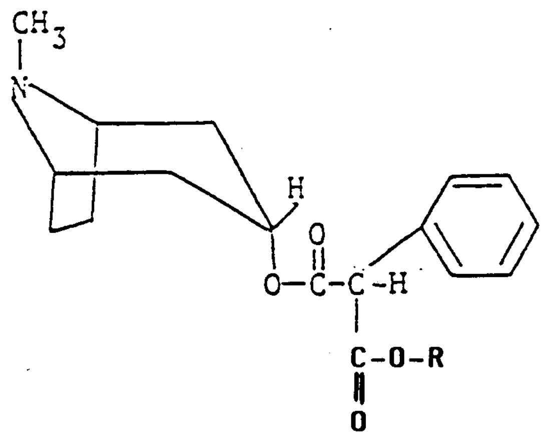

The observed pseudo first order hydrolytic rate constants (K) were half lives (t 1/2) in 0.01 M sodium hydroxide at pH 12 and 37°C

| Compound No. | R | K ($min^{-1}$)[a] S.E.M. | t 1/2 (min) |
|---|---|---|---|
| 4 | 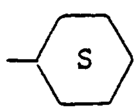 | 3.79 ± 0.8 x $10^{-5}$ | 182 |
| 6[b] | 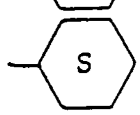 | 5.43 ± 0.1 x $10^{-1}$ | 1.28 |
| 7 | H | 19.8 ± 0.3 x $10^{-4}$ | 350 |

a) Average of three runs.

b) The quaternary iodide salt of the ester 4.

EXAMPLE 15

Determination of the enzymatic hydrolytic cleavage rates in human plasma at 37 °C

Freshly collected plasma was used which contained about 80% plasma diluted with anticoagulant citrate phosphate dextrose solution, U.S.P.

A 100 µl volume of a freshly prepared solution of the compound in methanol was added to 10 ml plasma, previously equilibrated a 37 °C in a water bath, and mixed thoroughly to result in an initial concentration of 6 x $10^{-3}$ mol. $liter^{-1}$. One ml samples of plasma were withdrawn from the test medium, mixed immediately with 3 ml ice cold acetonitrile, centrifuged and the supernatant analyzed by HPLC. The

first order hydrolytic rate constant was determined by following the disappearance of the compound as function of time. The results are given in Table 3.

TABLE 3

The observed first order hydrolytic rate constants (K) and half lives (t 1/2) in human plasma at 37°C

| Compound No. | R | K ($min^{-1}$) ± S.E.M. | t 1/2 (hr) |
|---|---|---|---|
| 2 | $-CH_2CH_3$ [a] | 15.0 ± 0.3 x $10^{-5}$ | 77.4 |
| 3 | 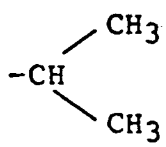 | 6.27 ± 0.3 x $10^{-5}$ | 185.5 |
| 4 | 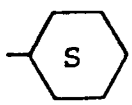 | 4.44 ± 0.4 x $10^{-5}$ | 269.8 |
| 5 | $-CH_2CH_3$ [b] | 14.1 ± 0.6 x $10^{-5}$ | 82.2 |
| 6 | 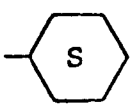 [c] | 6.31 ± 0.8 x $10^{-5}$ | 183.2 |

a) Average of 4 runs, the rest of data are average of 3 runs.

b) The quaternary iodide salt of the ester 2.

c) The quaternary iodide salt of the ester 4.

EXAMPLE 16

Determination of the rate of hydrolysis of tertiary ethyl ester (2) and rate of formation of its degradation product (7) in 0.01 M NaOH at pH 12 and 37°C

The mobile phase used for the separation of compound (2) and its degradation product, the half ester (7) consisted of 32% MeCN, 0.01M $KH_2PO_4$, 0.004M 1-octanesulfonic acid sodium salt and 0.1% acetic acid. At flow rate of 1.5 ml/min (2) and (7) have retention times of 9 min and 4.6 min, respectively.

Procedure

100 microliters of a freshly prepared solution of compound (2) in methanol was added to 10 ml buffered solution (pH 12), previously equilibrated to 37°C in a water bath to result in an initial concentration of 6 x $10^{-3}$ mol liter$^{-1}$. Samples of 1 ml were taken at time intervals and mixed with 3 ml of acetonitrile. 100

microliters of the collected samples were injected and disappearance of (2) and formation of (7) was followed by HPLC.

The results are given in Table 4.

TABLE 4

| Rate of hydrolysis of tertiary ethyl ester (2) to the half ester (7) in 0.01 N NaOH pH at 37°C | | |
|---|---|---|
| Compound No. | $K_{obsd}(min^{-1}) \pm$ S.E.M. | t 1/2 (min) |
| 2 | $16.5 \pm 0.58 \times 10^{-2}$ | $4.19 \pm 0.14$ |
| 7 | $1.68 \pm 0.34 \times 10^{-1}$ | $3.79 \pm 0.66$ |

## EXAMPLE 17

### Rate of hydrolysis of tropanyl hydrogen phenyl malonate (7) to the dibasic acid (phenylmalonic acid) in 0.01 N sodium hydroxide, pH 12 at 37°C

The previously described method for the determination of rate of hydrolysis of compound (2) was followed for the determination of hydrolysis of compound (7).

The disappearance of compound (7) was followed by HPLC.

The concentration of the formed dibasic acid was determined by HPLC. 15% acetonitrile, 0.01M $KH_2PO_4$ and 0.1% acetic acid was used as a mobile phase. At a flow rate of 2 ml/min, the product has a retention time of 4 min.

The collected samples after determination of rate of disappearance of compound (7) were used for the determination of rate of formation of the hydrolysis product (8). 100 microliters of the sample was injected and the formation of the product was followed by HPLC.

The results are given in Table 5.

TABLE 5

| Compound No. | $K_{obsd}(min^{-1}) \pm$ S.E.M. | t 1/2 (min) |
|---|---|---|
| 7 | $20.9 \pm 0.27 \times 10^{-4}$ | $331.25 \pm 4.31$ |
| 8 | $21.7 \pm 0.95 \times 10^{-4}$ | $316.01 \pm 33.5$ |

## EXAMPLE 18

### Determination of the Enzymatic Hydrolysis of the New Esters in Rat Liver Homogenate at 37°C

The liver homogenate was prepared by the following method. Two Sprague-Dawley rats were killed by decapitation, and the livers were removed, weighed and homogenized in a tissue homogenizer in 0.11 M aqueous phosphate buffer, pH 7.4, to make 20% liver homogenate. The homogenate was centrifuged and the supernatant was used for the test. 100 l of 0.6 M solution of the ester in methanol was mixed with 10 ml of the homogenate, previously equilibrated to 37°C in a water bath, to result in an initial concentration of 6 x $10^{-3}$ mol/liter$^{-1}$. samples of 1.0 ml were withdrawn at time intervals from the medium, added immediately to 3 ml ice-cold acetonitrile, shaken vigorously, and placed in a freezer. When all samples had been collected, they were centrifuged, and each supernatant was analyzed by HPLC. The results are shown in Table 6.

## TABLE 6

Rates of hydrolysis in liver homogenate of selected tropanyl esters

| Compound No. | R | K ($min^{-1}$) ± S.E. | t 1/2 (min) |
|---|---|---|---|
| 2 | $-CH_2CH_3$[a] | 1.99 ± 0.996 x $10^{-1}$ | 3.48 |
| 3 | 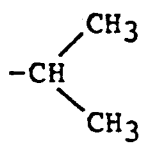 | 97.2 ± 0.76 x $10^{-3}$ | 7.12 |
| 4 | 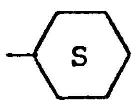 | 37.0 ± 0.3 x $10^{-3}$ | 18.73 |
| 5 | $-CH_2CH_3$[b] | 10.5 ± 0.28 x $10^{-2}$ | 6.6 |
| 6 | 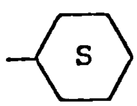 [c] | 10.4 ± 0.23 x $10^{-3}$ | 66.8 |

a) Mobile phase used for Compound 2, 3, and 5 was 32% acetonitrile, 0.01M $KH_2PO_4$, 0.004M 1-octane sulfonic acid sodium salt and 0.1% HAC. Mobile phase used for compound 4 and 6 was 40% acetonitrile, 0.01M $KH_2PO_4$, 0.004M 1-octane-sulfonic acid sodium salt and 0.1% HAC.

b) It is 2 - methiodide.

c) It is 4 - methiodide.

## EXAMPLE 19

### Pharmaceutical Study of the New Tropanyl Esters

The anticholinergic activity of all the new compounds was investigated *in vitro* by studying the inhibitory action of the drugs on carbachol-induced spasms of strips of ileum from freshly killed guinea pigs. The terminal ileum from guinea pig (200-500 g) was cut into 2-3 cm strips and suspended in freshly prepared Tyrode's solution in 40 ml organ bath. The solution was aerated with 95% oxygen and 5% carbon dioxide at 37 ° C. A tension of 1 g was applied to the tissue, and cumulative-response curves recorded with carbachol

until constant responses were obtained. A concentration of the antagonist was then added to the bath and after 10 seconds, further concentration response curves to carbachol were established. This procedure was then repeated using increasing concentrations of the antagonist. $EC_{50}$ was calculated for carbachol alone and for carbachol in the presence of the various antagonist concentrations. $PA_2$ value for each tested compound is listed in Table 7. Atropine was used as standard for comparison. The $PA_2$ was calculated as follows:

$PA_2 = \log (\frac{A}{a} - 1) - \log [\text{antagonist}]$

A = $EC_{50}$ or carbachol in presence of the antagonist
a = $EC_{50}$ for carbachol alone before adding antagonist

TABLE 7

| Compound No.[a] | R | $PA_2$ |
|---|---|---|
| Atropine | | 8.29 |
| 1 | $-CH_3$ | 6.55[b] |
| 2 | $-CH_2CH_3$ | 6.72 |
| 3 | 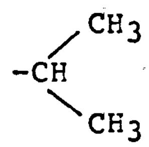 | 7.55 |
| 4 | 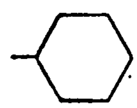 | 6.59[b] |
| 5[c] | $-CH_2CH_3$ | 7.86 |
| 6[d] | 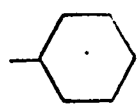 | 7.35 |
| 7 | H | 6.20 |

a) These results were average of two experiments.
b) Average of three experiments.
c) Quaternary iodide salt of tertiary ethyl ester.
d) Quaternary iodide salt of cyclohexyl ester.

EXAMPLE 20

Synthesis of isopropyl (±)-α-(carboxy)-8-methyl-8-azabicyclo[3•2•1]oct-3-yl-benzeneacetate methiodide

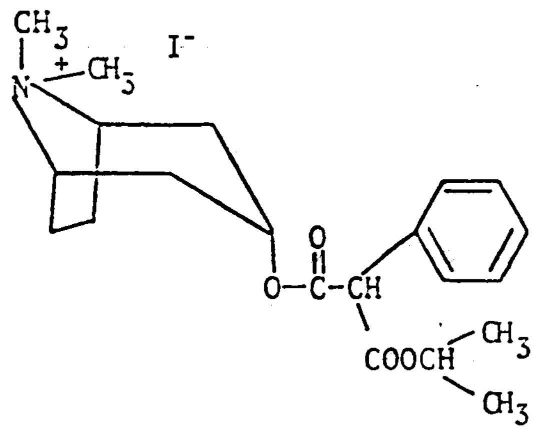

To a solution of 2.07 g (0.006 mol) of isopropyl (±)-α-(carboxy)-8-methyl-8-azabicyclo-[3•2•1]oct-3-1-benzeneacetate in 20 ml of dry benzene was added 6 ml methyl iodide and the mixture was stirred at room temperature for 6 hr. The yellowish white solid product was filtered, dried and crystallized from ethanol-ether mixture to yield 1.8 g (61.56%) of the title compound m.p. 251-254 °C.
$^1H$ NMR (DMSO-$d_6$) δ 7.3 (s, 5H, $C_6H_5$), 4.96 (s, 3H $C_3$ of tropine + $-COOC\underline{H}(CH_3)_2$ +

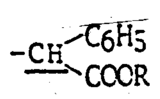 );

3.16-1.97 [m, 14H, $N^+CH_3$ (s, 3.16) + $N^+CH_3$ (s, 3.06) + bicyclic envelop of tropine]; 1.27 (br s, 6H,

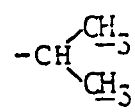 .

| Anal. for $C_{21}H_{30}INO_4$.0.5 $H_2O$ | | | |
|---|---|---|---|
| | C% | H% | N% |
| Theoretical | 50.81 | 6.29 | 2.82 |
| Found | 50.67 | 6.25 | 2.95 |

The pharmacological result: $PA_2$ = 7.61.

EXAMPLE 21

The mydriatic activity of the compounds is illustrated by the following procedure.

The mydriatic activity of the soft ester drugs (Compound 5) was evaluated in 0.9% saline solution at concentrations of 0.05% and 0.1% compared to 0.1% tropicamide (Mydriacyl®). Normal New Zealand albino rabbits of either sex, weighing about 2 kg were employed. The animals were placed in wooden restraining boxes. Standard doses of 50 µl were applied to the rabbits' eyes and pupillary changes were measured in a light and temperature controlled room. The degree of pupil dilation, in millimeters, was measured with a Starrett micrometer held at a constant distance at time intervals of 15, 30, 60 90, 120, 180, 240, 300 and 360 minutes. The differences in the same animals between the pupil diameter of the eye with

drug versus the other eye with saline was recorded. Each point on the graph is the average obtained on 3-6 animals. The recovery time is defined as that time required for the pupil diameter to return to 1.0 millimeter in diameter.

The results are set forth in Fig. 1 which depicts mydriasis as a function of time. Tropicamide is typical of the shortest acting of the commercially available anticholinergic mydriatic agents. The active agent therein is tropicamide - chemical name = Benzeneacetamide N-ethyl-α-(hydroxymethyl)-N-(4-pyridinylmethyl)-. As is apparent from the results depicted in Fig. 1, the "recovery time", i.e., recovery from mydriasis, is much shorter with the compositions and methods of the invention than with the commercially available products.

Tables 8, 9 and 10 below set forth yield and analysis data for compounds depicted therein prepared according to the above-described procedures:

## TABLE 8

OXALATE SALTS OF SOFT ANTICHOLINERGIC AGENTS

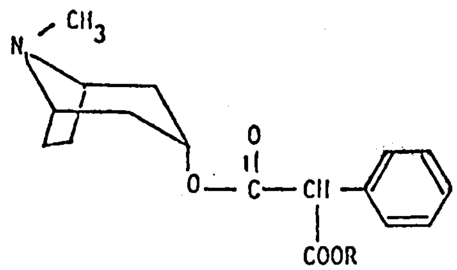

· $C_2H_2O_4$

| Compd | R | m.p.[a] °C | Yield % | Formula | Calculated C% | Calculated H% | Calculated N% | Found C% | Found H% | Found N% |
|---|---|---|---|---|---|---|---|---|---|---|
| II | H | 112-115 | 23 | $C_{17}H_{21}NO_4 \cdot C_2H_2O_4$ 0.5$H_2O$ | 56.71 | 6.01 | 3.48 | 56.94 | 6.26 | 3.32 |
| Va | $CH_3$ | 89-91 | 64.5 | $C_{18}H_{23}NO_4 \cdot C_2H_2O_4 \cdot H_2O$ | 56.47 | 6.39 | 3.29 | 56.48 | 6.2 | 2.94 |
| Vb | $CH_2CH_3$ | 135-137 | 75 | $C_{19}H_{25}NO_4 \cdot C_2H_2O_4$ | 59.85 | 6.45 | 3.32 | 59.88 | 6.52 | 3.3 |
| Vc | $CH(CH_3)_2$ | 78-80 | 72.4 | $C_{20}H_{27}NO_4 \cdot C_2H_2O_4$ | 60.68 | 6.71 | 3.21 | 60.41 | 6.77 | 3.13 |
| Vd | $C_6H_{11}$ | 160-162 | 77.91 | $C_{23}H_{31}NO_4 \cdot C_2H_2O_4$ | 63.15 | 6.99 | 2.94 | 63.05 | 7.26 | 2.78 |

[a] All compounds were recrystallized from methanol-ether.

TABLE 9

QUATERNARY IODIDES

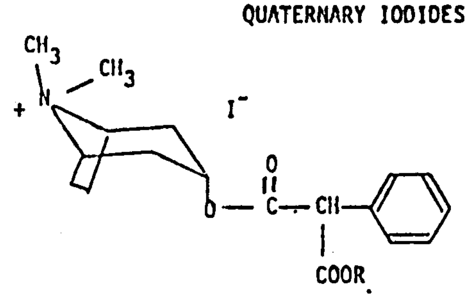

| Compd | R | m.p. °C | Cryst. Solvent | Yield % | Formula | Calculated C | Calculated H | Calculated N | Found C | Found H | Found N |
|---|---|---|---|---|---|---|---|---|---|---|---|
| VIb | $-CH_2CH_3$ | 232-234 (dec) | methanol-ether | 50.9 | $C_{20}H_{28}INO_4$ | 50.75 | 5.92 | 2.96 | 50.55 | 5.97 | 2.97 |
| VIc | $-CH(CH_3)_2$ | 251-254 | ethanol-ether | 61.56 | $C_{21}H_{30}INO_4$ $0.5H_2O$ | 50.81 | 6.29 | 2.82 | 50.67 | 6.26 | 2.95 |
| VId | $C_6H_{11}$ | 228-230 | methanol-ether | 55.9 | $C_{24}H_{34}INO_4$ $0.25H_2O$ | 54.19 | 6.53 | 2.63 | 54.00 | 6.48 | 2.50 |

TABLE 10

QUATERNARY DIMETHYLSULFATES

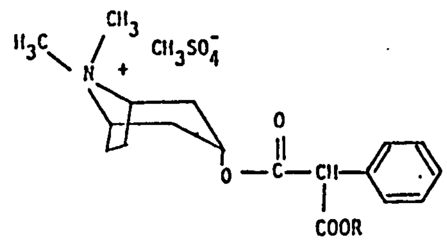

| Compd | R | m.p. °C | Cryst. Solvent | Yield % | Formula | Calculated C | Calculated H | Calculated N | Calculated S | Found C | Found H | Found N | Found S |
|---|---|---|---|---|---|---|---|---|---|---|---|---|---|
| VIIa | $CH_3$ | 138-140 | Methanol/Ether | 22 | $C_{20}H_{29}NO_8S\cdot\frac{1}{2}H_2O$ | 53.08 | 6.68 | 3.09 | 7.08 | 53.06 | 6.65 | 3.01 | 7.14 |
| VIIb | $C_2H_5$ | 165-167 | Methanol/Ether | 62 | $C_{21}H_{31}NO_8S$ | 55.13 | 6.83 | 3.06 | 7.01 | 55.10 | 6.87 | 2.96 | 7.02 |
| VIIc | $CH(CH_3)_2$ | 128-130 | Methanol/Ether | 48 | $C_{22}H_{33}NO_8S$ | 56.03 | 7.05 | 2.97 | 6.80 | 55.91 | 7.08 | 2.96 | 6.78 |
| VIId | $C_6H_{11}$ | 196-198 | Methanol/Ether | 36 | $C_{25}H_{37}NO_8S\cdot\frac{1}{2}H_2O$ | 57.67 | 7.36 | 2.69 | 6.16 | 57.91 | 7.15 | 2.68 | 6.17 |

Table 11 sets forth hydrolysis kinetic data in various media for several compounds according to the invention.

TABLE 11

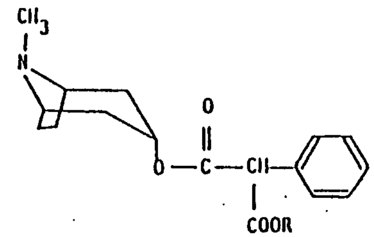

HYDROLYSIS KINETICS IN AQUEOUS SOLUTION (pH 12), HUMAN PLASMA (37°C), AND RAT LIVER HOMOGENATE (37°C)

| COMPOUND | R | $t_{1/2}$ (Min) AQUEOUS SOLN, pH 12[a], 37°C ($k_{obsd}$ $min^{-1}$) | $t_{1/2}$ (h) HUMAN PLASMA, 37°C[a] ($k_{obsd}$ $min^{-1}$) | $t_{1/2}$ (Min) RAT LIVER HOMOGENATE, 37°C[a] ($k_{obsd}$ $min^{-1}$) |
|---|---|---|---|---|
| II | H | 350 (19.8 ± 0.3 x $10^{-4}$) | — | — |
| Va | Me | 4.80 (1.43 ± 0.12 x $10^{-1}$) | — | — |
| Vb | Et | 4.08 (16.9 ± 0.15 x $10^{-2}$) | 77.4 (15.0 ± 0.3 x $10^{-5}$) | 3.48 (1.99 ± 0.996 x $10^{-1}$) |
| Vc | i-Pr | 22.8 (30 ± 0.77 x $10^{-3}$) | 185.5 (6.27 ± 0.3 x $10^{-5}$) | 7.12 (97.2 ± 0.76 x $10^{-3}$) |
| Vd | c-Hexyl | 182 (379 ± 0.8 x $10^{-5}$) | 269.8 (4.44 ± 0.4 x $10^{-5}$) | 18.7 (37.0 ± 0.3 x $10^{-3}$) |
| VIb | Et ($N^+$)[b] | 0.85 (80.8 ± 0.75 x $10^{-2}$) | 82.2 (14.1 ± 0.6 x $10^{-5}$) | 6.6 (10.5 ± 0.28 x $10^{-2}$) |
| VId | c-Hexyl ($N^+$)[b] | 1.28 (5.43 ± 0.1 x $10^{-1}$) | 183.2 (6.31 ± 0.8 x $10^{-5}$) | 66.8 (10.4 ± 0.23 x $10^{-3}$) |

[a] Average of three runs ± SEM.

[b] Tests were performed with the quaternary methiodide salts.

Table 12 sets forth anticholinergic activities for various of the compounds according to the present invention. See Table 11 for the identities of the compounds identified by Roman numerals. The activities were obtained according to the method described in Example 19.

TABLE 12

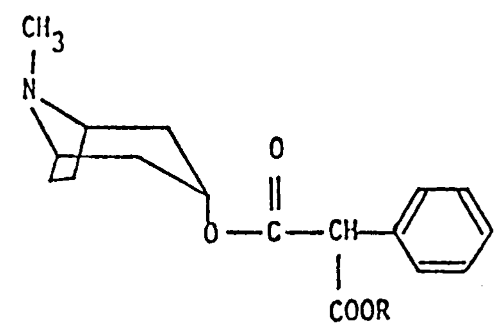

ANTICHOLINERGIC ACTIVITY OF SOFT ATROPINE AGENTS

| SOFT DRUG R | $PA_2$ TERTIARY AMINE | $PA_2$ QUATERNARY AMINE[a] |
|---|---|---|
| H | 6.20 (II) | ——— |
| Me | 6.55 (Va) | ——— |
| Et | 6.72 (Vb) | 7.85 (VIb) |
| i-Pr | 7.55 (Vc) | 7.61 (VIc) |
| c-Hexyl | 6.59 (Vd) | 7.35 (VId) |
| Atropine | 8.29 | ——— |

[a] Tests were carried out with the quaternary methiodide soft esters.

## Claims

**Claims for the following Contracting States : BE, CH, DE, FR, GB, IT, LI, LU, NL, SE**

**1.** A compound having the formula

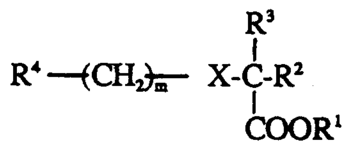 (I)

wherein:

$R^1$ is a staight or branched chain alkyl or cycloalkyl group having up to 8 carbon atoms;
$R^2$ is an aryl, cycloalkyl or alkyl group having up to 8 carbon atoms;
$R^3$ is H or a group defined by $R^2$,
X is

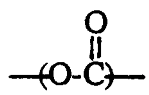 or 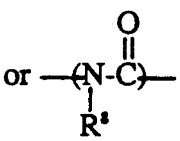

wherein $R^8$ is H or a straight or branched chain alkyl group having up to 5 carbon atoms;
m is an integer from 0 to 4; and
$R^4$ is

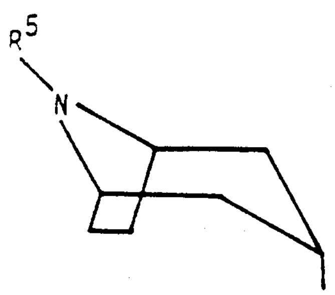 or 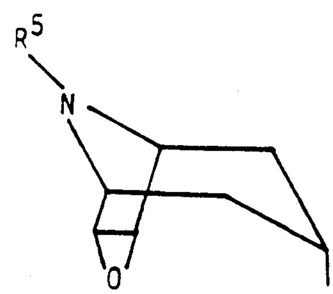

wherein $R^5$ is an alkyl group having up to 5 carbon atoms; or $R^4$ is

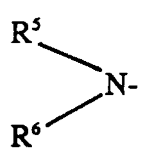

wherein $R^5$ and $R^6$ may be the same or different and are alkyl groups having up to 5 carbon atoms; or $R^4$ is an ortho-, meta- or para-substituted alkyl pyridine of the formula

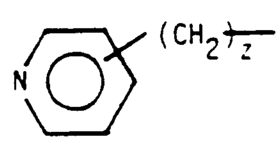

wherein Z is an integer from 0 to 3; or a quaternary ammonium salt of a compound of formula (I) with a compound of the formula $R^7Y$ wherein $R^7$ is an alkyl group having up to 5 carbon atoms and Y is a pharmaceutically acceptable anion.

**2.** A compound according to Claim 1 wherein $R^2$ is phenyl.

**3.** A compound according to Claim 1 wherein $R^3$ is H.

**4.** A compound according to Claim 1 having the formula

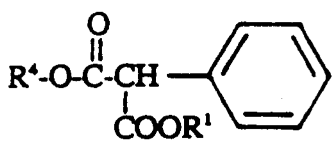

(Ia)

wherein $R^1$ is a straight or branched chain alkyl or cycloalkyl group having up to 8 carbon atoms; and $R^4$ is

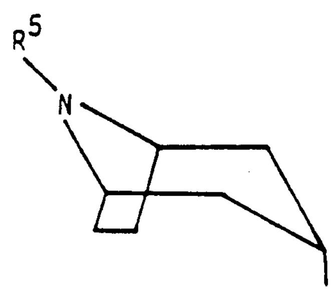

or

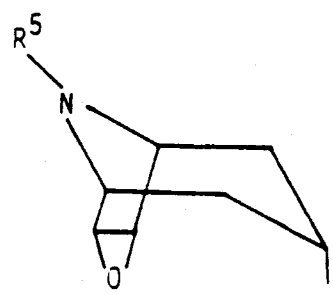

wherein $R^5$ is an alkyl group having up to 5 carbon atoms; or a quaternary ammonium salt of a compound of formula (Ia) with a compound of the formula $R^7Y$ wherein $R^7$ is an alkyl group having up to 5 carbon atoms and Y is a pharmaceutically acceptable anion.

**5.** A compound according to any one of Claims 1 to 4 wherein $R^1$ is a cycloalkyl group.

**6.** A compound according to any one of Claims 1 to 4 wherein $R^1$ is a straight or branched chain alkyl group.

**7.** A compound according to Claim 6 wherein $R^1$ is methyl, ethyl or propyl.

**8.** A quaternary ammonium compound according to any one of Claims 1 to 7 wherein $R^7$ is methyl and Y is halide, sulfate, alkylsulfate or alkylsulfonate.

9. A compound according to Claim 1 having the formula

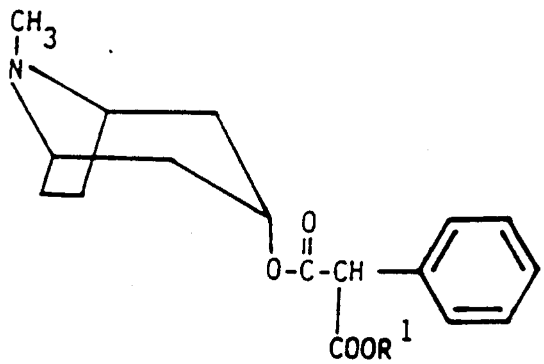

wherein $R^1$ is as defined in Claim 1.

10. A compound according to Claim 9 wherein $R^1$ is a cycloalkyl group.

11. A compound according to Claim 10 wherein $R^1$ is cyclohexyl.

12. A compound according to Claim 9 wherein $R^1$ is a straight or branched chain alkyl group.

13. A compound according to Claim 12 wherein $R^1$ is methyl.

14. A compound according to Claim 12 wherein $R^1$ is ethyl.

15. A compound according to Claim 12 wherein $R^1$ is isopropyl.

16. A compound according to any one of Claims 9 to 15, in the form of its salt with dimethylsulfate.

17. A compound according to Claim 1 having the formula

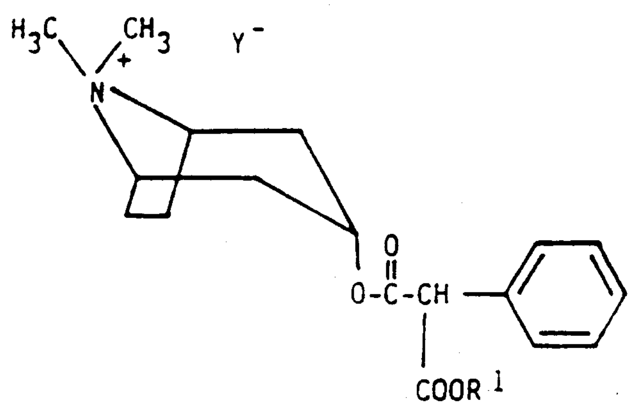

wherein $R^1$ and Y are as defined in Claim 1.

18. A compound according to Claim 17 wherein $R^1$ is a cycloalkyl group.

19. A compound according to Claim 18 wherein $R^1$ is cyclohexyl.

20. A compound according to Claim 17 wherein $R^1$ is a straight or branched chain alkyl group.

21. A compound according to Claim 20 wherein $R^1$ is methyl.

**22.** A compound according to Claim 20 wherein $R^1$ is ethyl.

**23.** A compound according to Claim 20 wherein $R^1$ is isopropyl.

**24.** A compound according to any one of Claims 17 to 23, wherein $Y^-$ is $I^-$ or $CH_3SO_4^-$.

**25.** A compound according to Claim 1 having the formula

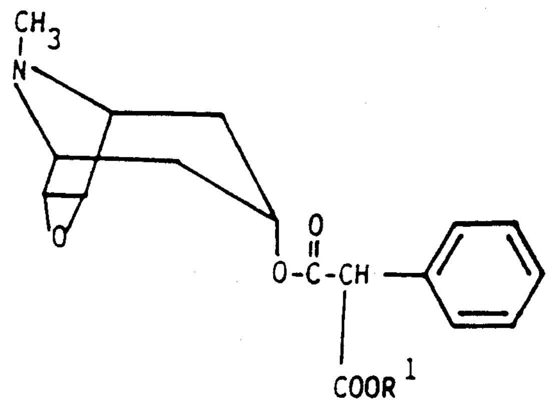

wherein $R^1$ is as defined in Claim 1.

**26.** A compound according to Claim 1 having the formula

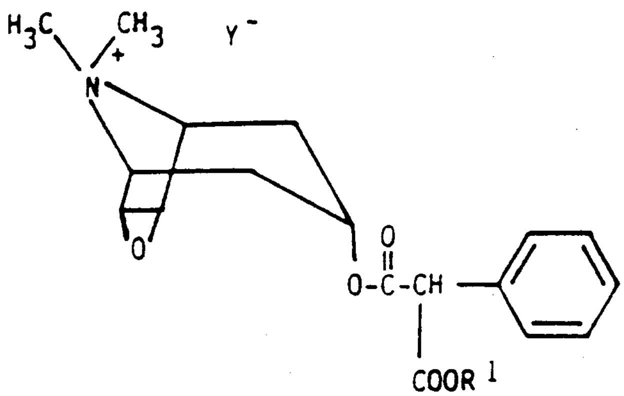

wherein $R^1$ and Y are as defined in Claim 1.

**27.** A pharmaceutical composition in unit dosage form comprising an anticholinergic effective amount of a compound as claimed in any one of Claims 1 to 26 and a pharmaceutically acceptable carrier therefor.

**28.** A pharmaceutical composition in unit dosage form for administration to the eye or eyes of an animal or human comprising a mydriatic effective amount of a compound as claimed in any one of Claims 1 to 26 and a pharmaceutically acceptable carrier therefor.

**Claims for the following Contracting States : AT, ES, GR**

**1.** Process for producing a compound having the formula

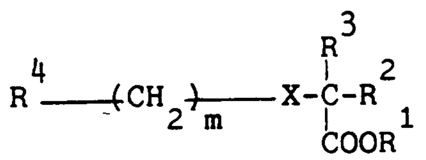

(I)

wherein

- $R^1$ is a straight or branched chain alkyl or cycloalkyl group having up to 8 carbon atoms;
- $R^2$ is an aryl, cycloalkyl or alkyl group having up to 8 carbon atoms;
- $R^3$ is H or a group defined by $R^2$;
- X is

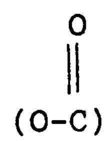 or 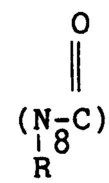

wherein $R^8$ is H or a straight or branched chain alkyl group having up to 5 carbon atoms;

- m is an integer from 0 to 4; and
- $R^4$ is

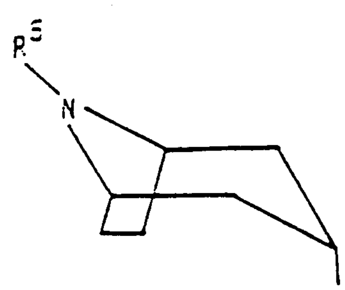

or

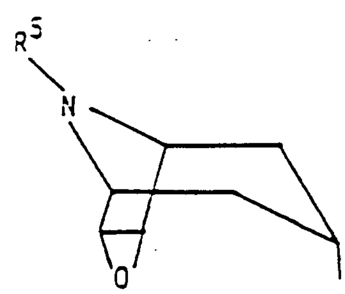

wherein $R^5$ is an alkyl group having up to 5 carbon atoms; or $R^4$ is

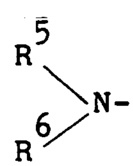

wherein $R^5$ and $R^6$ may be the same or different and are alkyl groups having up to 5 carbon atoms; or $R^4$ is an ortho-, meta- or para-substitued alkyl pyridine of the formula

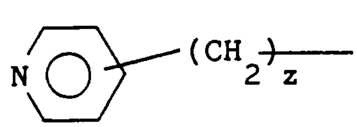

wherein z is an integer from 0 to 3; or a quaternary ammonium salt of a compound of formula (I) with a compound of the formula $R^7Y$ wherein $R^7$ is an alkyl group having up to 5 carbon atoms and Y is a pharmaceutically acceptable anion, the steps comprising reacting a compound of the formula II

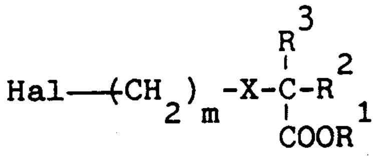

(II)

wherein
$R^1$, $R^2$, $R^3$, X and m have the same meaning as in formula I and Hal is a halogen, with a compound of formula $R^4OH$, wherein $R^4$ has the same meaning as in formula I, in a suitable solvent and, possibly, reacting the resulting compound of formula I with a suitable quaternary agent of the formula $R^7Y$ wherein $R^7$ is an alkyl group having up to 5 carbon atoms and Y is a pharmaceutically acceptable anion.

**2.** A process according to claim 1 wherein in formula II $R^2$ is phenyl.

**3.** A process according to claim 1 wherein in formula II $R^3$ is H.

**4.** A process according to claim 1 wherein in formula II $R^1$is a straight or branched chain alkyl or cycloalkyl group having up to 8 carbon atoms; and wherein in formula $R^4OH$
$R^4$ is

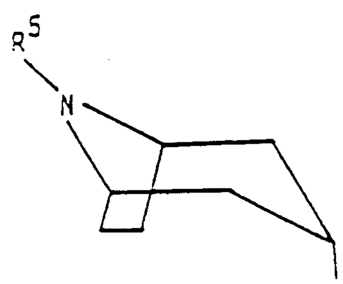

or

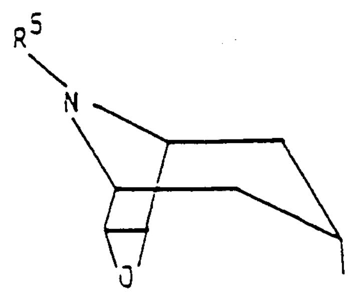

wherein $R^5$ is an alkyl group having up to 5 carbon atoms.

**5.** A process according to any one of claims 1 to 4 wherein in formula II $R^1$ is a cycloalkyl group.

**6.** A process according to any one of claims 1 to 4 wherein in formula II $R^1$ is a straight or branched chain alkyl group.

**7.** A process according to claim 6 wherein in formula II $R^1$ is methyl, ethyl or propyl.

**8.** A process according to any of claims 1 to 7 wherein in formula $R^7Y$ $R^7$ is methyl and Y is halide, sulfate, alkylsulfate or alkylsulfonate.

**9.** A process according to claim 1 wherein in formula II $R^2$ is phenyl, $R^3$ is H, m is 0 and in formula $R^4OH$
$R^4$ is

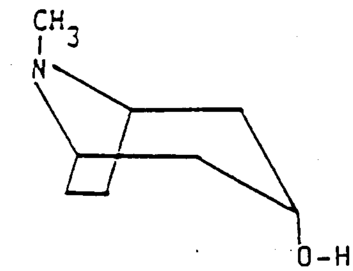

10. A process according to claim 9 wherein in formula II $R^1$ is a cycloalkyl group.

11. A process according to claim 10 wherein in formula II $R^1$ is cyclohexyl.

12. A process according to claim 9 wherein in formula II $R^1$ is a straight or branched chain alkyl group.

13. A process according to claim 12 wherein in formula II $R^1$ is methyl.

14. A process according to claim 12 wherein in formula II $R^1$ is ethyl.

15. A process according to claim 12 wherein in formula II $R^1$ is isopropyl.

16. A process according to any one of claims 9 to 15 by reacting the resulting product of formula I with dimethylsulfate to the corresponding quaternary ammonium salt.

**Patentansprüche**

**Patentansprüche für folgende Vertragsstaaten : BE, CH, DE, FR, GB, IT, LI, LU, NL, SE**

1. Eine Verbindung der Formel

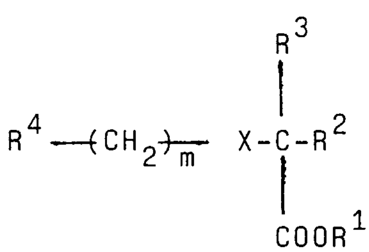

(I)

worin $R^1$ eine gerad- oder verzweigtkettige Alkyl- oder Cycloalkylgruppe mit bis zu 8 Kohlenstoffatomen ist;
$R_2$ ein Aryl-, Cycloalkyl- oder Alkylgruppe mit bis zu 8 Kohlenstoffatomen ist;
$R^3$ H oder eine durch $R^2$ definierte Gruppe ist;
X

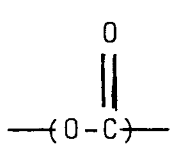 oder 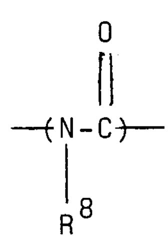

ist, worin $R^8$ H oder eine gerad- oder verzweigtkettige Alkylgruppe mit bis zu 5 Kohlenstoffatomen ist; m eine ganze Zahl von 0 bis 4 ist; und
$R^4$

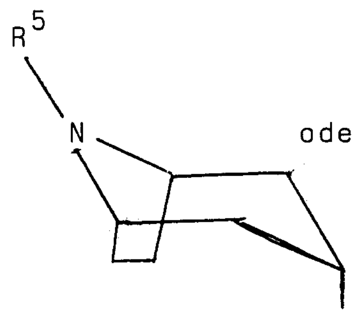

oder

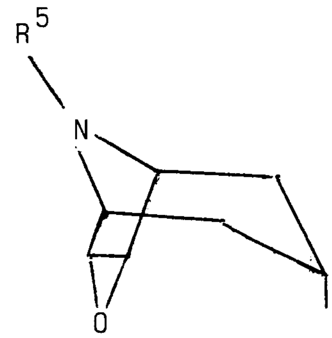

ist, worin $R^5$ eine Alkylgruppe mit bis zu 5 Kohlenstoffatomen ist; oder $R^4$

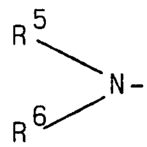

ist, worin $R^5$ und $R^6$ gleich oder verschieden sein können und Alkylgruppen mit bis zu 5 Kohlenstoffatomen sind;
oder $R^4$ ein ortho-, meta- oder parasubstituiertes Alkylpyridin der Formel

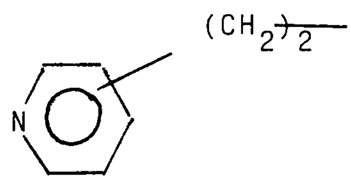

ist, worin Z eine ganze Zahl von 0 bis 3 ist; oder ein quartäres Ammoniumsalz einer Verbindung der Formel (I) mit einer Verbindung der Formel $R^7Y$, worin $R^7$ eine Alkylgruppe mit bis zu 5 Kohlenstoffatomen und Y ein pharmazeutisch verträgliches Anion ist.

**2.** Eine Verbindung nach Anspruch 1, worin $R^2$ Phenyl ist.

**3.** Eine Verbindung nach Anspruch 1, worin $R^3$ H ist.

**4.** Eine Verbindung nach Anspruch 1 der Formel

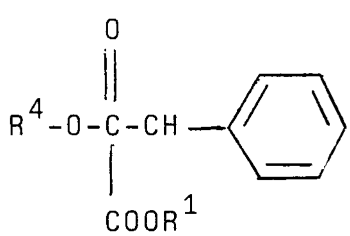

(Ia),

worin $R^1$ eine gerad- oder verzweigtkettige Alkyl- oder Cycloalkylgruppe mit bis zu 8 Kohlenstoffatomen

ist; und
$R^4$

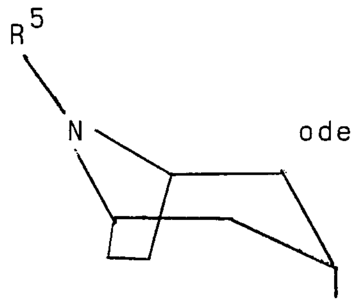

oder

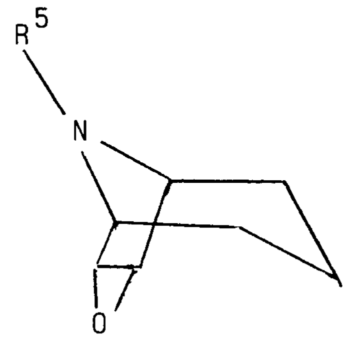

ist, worin $R^5$ eine Alkylgruppe mit bis zu 5 Kohlenstoffatomen ist; oder ein quartäres Ammoniumsalz einer Verbindung der Formel (Ia) mit einer Verbindung der Formel $R^7Y$, worin $R^7$ eine Alkylgruppe mit bis zu 5 Kohlenstoffatomen und Y ein pharmazeutisch verträgliches Anion ist.

**5.** Eine Verbindung nach einem der Ansprüche 1 bis 4, worin $R^1$ eine Cycloalkylgruppe ist.

**6.** Eine Verbindung nach einem der Ansprüche 1 bis 4, worin $R^1$ eine gerad- oder verzweigtkettige Alkylgruppe ist.

**7.** Eine Verbindung nach Anspruch 6, worin $R^1$ Methyl, Ethyl oder Propyl ist.

**8.** Eine quartäre Ammoniumverbindung nach der einem der Ansprüche 1 bis 7, worin $R^7$ Methyl und Y ein Halogenid, Sulfat, Alkylsulfat oder Alkylsulfonat ist.

**9.** Eine Verbindung nach Anspruch 1 der Formel

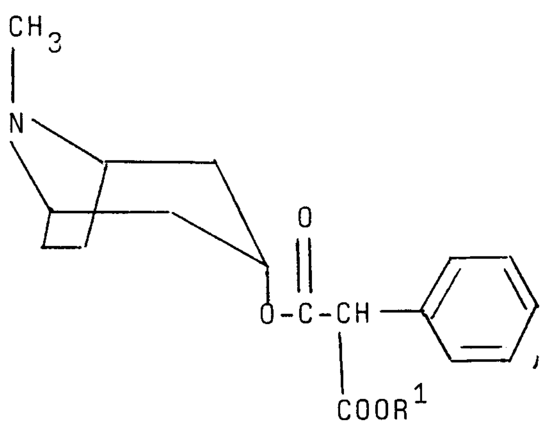

worin $R^1$ wie in Anspruch 1 definiert ist.

**10.** Eine Verbindung nach Anspruch 9, worin $R^1$ eine Cycloalkylgruppe ist.

**11.** Eine Verbindung nach Anspruch 10, worin $R^1$ Cyclohexyl ist.

**12.** Eine Verbindung nach Anspruch 9, worin $R^1$ eine gerad- oder verzweigtkettige Alkylgruppe ist.

**13.** Eine Verbindung nach Anspruch 12, worin $R^1$ Methyl ist.

**14.** Eine Verbindung nach Anspruch 12, worin $R^1$ Ethyl ist.

**15.** Eine Verbindung nach Anspruch 12, worin $R^1$ Isopropyl ist.

**16.** Eine Verbindung nach einem der Ansprüche 9 bis 15 in der Form seines Dimethylsulfatsalzes.

**17.** Eine ihrer Verbindung nach Anspruch 1 der Formel

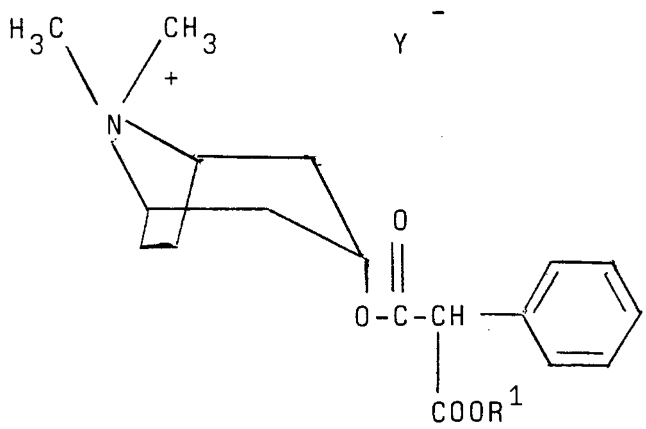

worin $R^1$ und Y wie in Anspruch 1 definiert sind.

**18.** Eine Verbindung nach Anspruch 17, worin $R^1$ eine Cycloalkylgruppe ist.

**19.** Eine Verbindung nach Anspruch 18, worin $R^1$ Cyclohexyl ist.

**20.** Eine Verbindung nach Anspruch 17, worin $R^1$ eine gerad- oder verzweigtkettige Alkylgruppe ist.

**21.** Eine Verbindung nach Anspruch 20, worin $R^1$ Methyl ist.

**22.** Eine Verbindung nach Anspruch 20, worin $R^1$ Ethyl ist.

**23.** Eine Verbindung nach Anspruch 20, worin $R^1$ Isopropyl ist.

**24.** Eine Verbindung nach einem der Ansprüche 17 bis 23, worin $Y^-$ gleich $I^-$ oder $CH_3SO_4^-$ ist.

**25.** Eine Verbindung nach Anspruch 1, der Formel

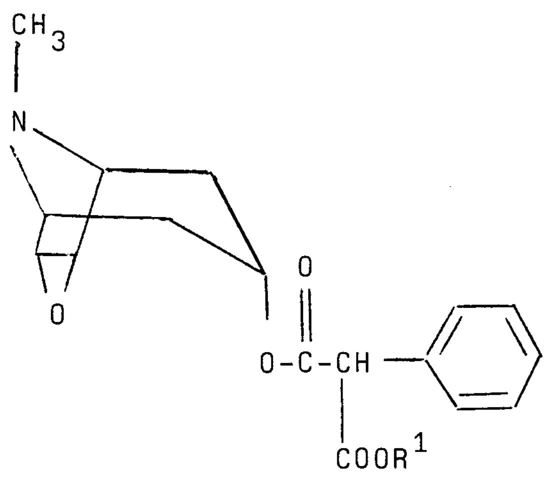

worin $R^1$ wie in Anspruch 1 definiert ist.

**26.** Eine Verbindung nach Anspruch 1 der Formel

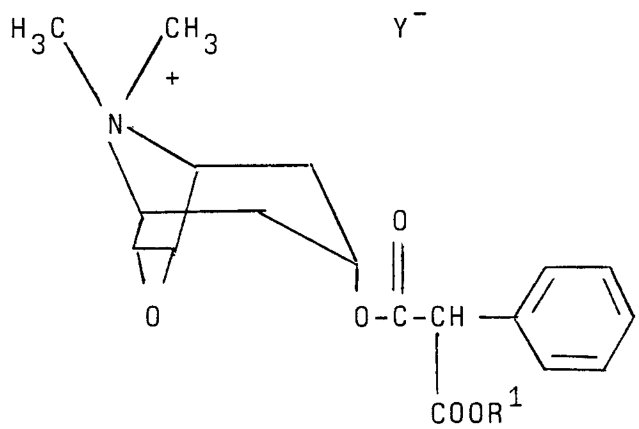

,

worin $R^1$ und Y wie in Anspruch 1 definiert sind.

**27.** Eine pharmazeutische Einzeldosis-Zubereitung, enthaltend eine anticholinergisch wirksame Menge einer Verbindung, wie in einem der Ansprüche 1 bis 26 beansprucht, und einen pharmazeutisch verträglischen Träger dafür.

**28.** Eine pharmazeutische Einzeldosis-Zubereitung zur Verabreichung an das Auge oder die Augen eines Tieres oder Menschen, enthaltend eine mydriatisch wirksame Menge einer Verbindung, wie in den Ansprüchen 1 bis 26 beansprucht, und einen pharmazeutisch verträglichen Träger dafür.

**Patentansprüche für folgende Vertragsstaaten : AT, ES, GR**

**1.** Ein Verfahren zur Herstellung einer Verbindung der Formel

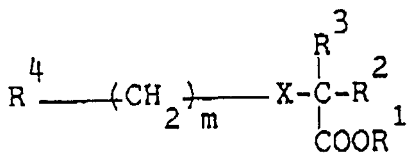

(I)

worin

$R^1$ eine gerad- oder verzweigtkettige Alkyl- oder Cycloalkylgruppe mit bis zu 8 Kohlenstoffatomen ist;

$R^2$ eine Aryl-, Cycloalkyl- oder Alkylgruppe mit bis zu 8 Kohlenstoffatomen ist;

$R^3$ H oder eine durch $R^2$ definierte Gruppe ist;

X

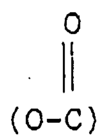

oder

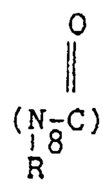

ist,
worin $R^8$ Wasserstoff oder eine gerad- oder verzweigtkettige Alkylgruppe mit bis zu 5 Kohlenstoffatomen ist;

m eine ganze Zahl von 0 bis 4 und
$R^4$

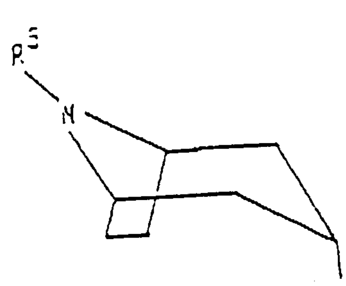

oder

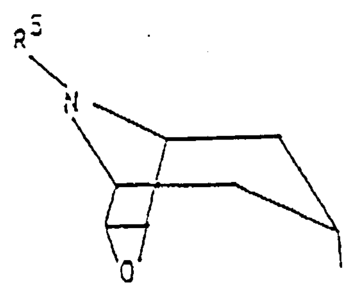

ist, worin $R^5$ eine Alkylgruppe mit bis zu 5 Kohlenstoffatomen ist;
oder $R^4$

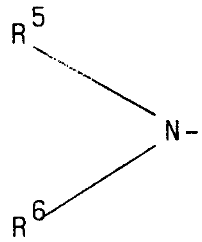

ist, worin $R^5$ und $R^6$ gleich oder verschieden sein können und Alkylgruppen mit bis zu 5 Kohlenstoffatomen sind
oder $R^4$ ein ortho-, meta- oder parasubstituiertes Alkylpyridin der Formel

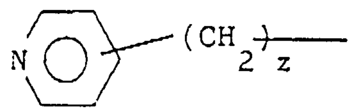

ist, worin z eine ganze Zahl von 0 bis 3; oder ein quartäres Ammoniumsalz einer Verbindung der Formel (I) mit einer Verbindung der Formel $R^7Y$, worin $R^7$ eine Alkylgruppe mit bis zu 5 Kohlenstoffatomen und Y ein pharmazeutisch verträgliches Anion ist, enthaltend die Umsetzung der Verbindung der Formel II

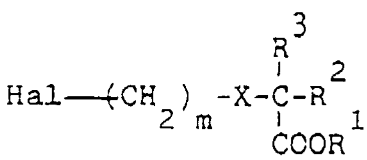 (II)

worin $R^1$, $R^2$, $R^3$, X und n dieselbe Bedeutung wie in Formel I haben und Hal Halogen ist, mit einer Verbindung der Formel $R^4OH$, worin $R^4$ dieselbe Bedeutung wie in Formel I hat, in einem geeigneten Lösungsmittel und gegebenenfalls Umsetzen der erhaltenen Verbindung der Formel I mit einem geeigneten quartären Mittel der Formel $R^7Y$, worin $R^7$ eine Alkylgruppe mit bis zu 5 Kohlenstoffatomen und Y ein pharmazeutisch verträgliches Anion ist.

**2.** Ein Verfahren nach Anspruch 1, worin in Formel II $R^2$ Phenyl ist.

**3.** Ein Verfahren nach Anspruch 1, worin in Formel II $R^3$ H ist.

**4.** Ein Verfahren nach Anspruch 1, worin in Formel II $R^1$ eine gerad- oder verzweigtkettige Alkyl- oder Cycloalkylgruppe mit bis zu 8 Kohlenstoffatomen ist und worin in Formel $R^4OH$
$R^4$

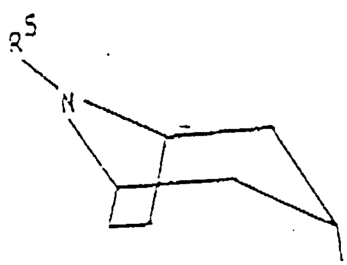 oder 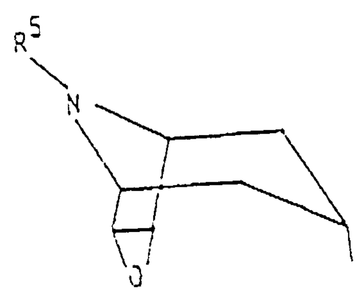

ist, worin $R^5$ eine Alkylgruppe mit bis zu 5 Kohlenstoffatomen ist.

5. Ein Verfahren nach einem der Ansprüche 1 bis 4, worin in Formel II $R^1$ eine Cycloalkylgruppe ist.

6. Ein Verfahren nach einem der Ansprüche 1 bis 4, worin in Formel II $R^1$ eine gerad- oder verzweigtkettige Alkylgruppe ist.

7. Ein Verfahren nach Anspruch 6, worin in Formel II $R^1$ Methyl, Ethyl oder Propyl ist.

8. Ein Verfahren nach einem der Ansprüche 1 bis 7, worin in Formel $R^7$ Y $R^7$ Methyl und Y Halogenid, Sulfat, Alkylsulfat oder Alkylsulfonat ist.

9. Ein Verfahren nach Anspruch 1, worin in Formel II $R^2$ Phenyl, $R^3$ H, m 0 und in Formel $R^4$OH $R^4$

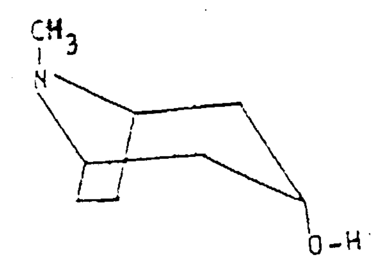

ist.

10. Ein Verfahren nach Anspruch 9, worin in Formel II $R^1$ eine Cycloalkylgruppe ist.

11. Ein Verfahren nach Anspruch 10, worin in Formel II $R^1$ Cyclohexyl ist.

12. Ein Verfahren nach Anspruch 9, worin in Formel II $R^1$ eine gerad- oder verzweigtkettige Alkylgruppe ist.

13. Ein Verfahren nach Anspruch 12, worin in Formel II $R^1$ Methyl ist.

14. Ein Verfahren nach Anspruch 12, worin in Formel II $R^1$ Ethyl ist.

15. Ein Verfahren nach Anspruch 12, worin in Formel II $R^1$ Isopropyl ist.

16. Ein Verfahren nach einem der Ansprüche 9 bis 15, worin das erhaltene Produkt der Formel I mit Dimethylsulfat zum korrespondierenden quartären Ammoniumsalz umgesetzt wird.

**Revendications**
**Revendications pour les Etats contractants suivants : BE, CH, DE, FR, GB, IT, LI, LU, NL, SE**

1. Composé de formule

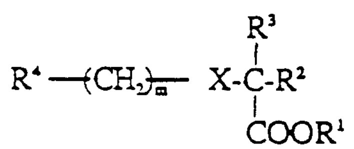

(I)

où:

$R^1$ est un groupe alkyle en chaîne droite ou ramifiée ou cycloalkyle ayant jusqu'à 8 atomes de carbone;

$R^2$ est un groupe aryle, cycloalkyle ou alkyle ayant juscu'à 8 atomes de carbone;

$R^3$ est H ou un groupe défini par $R^2$;

X est

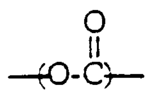

ou

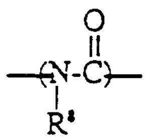

où $R^8$ est H ou un groupe alkyle en chaîne droite ou ramifiée ayant jusqu'à 5 atomes de carbone;
m est un entier de 0 à 4; et
$R^4$ est

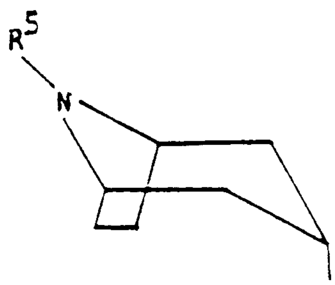

ou

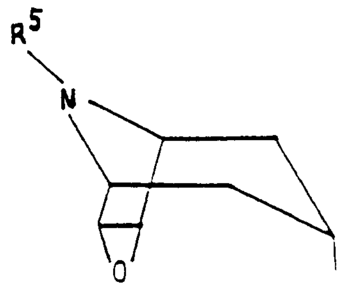

où $R^5$ est un groupe alkyle ayant jusqu'à 5 atomes de carbone; ou $R^4$ est

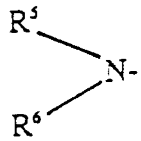

où $R^5$ et $R^6$ peuvent être identiques ou différents et sont des groupes alkyle ayant jusqu'à 5 atomes de carbone; ou $R^4$ est une alkyl pyridine ortho-, méta- ou para-substituée de formule

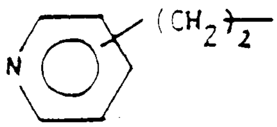

où Z est un entier de 0 à 3; ou un sel d'ammonium quaternaire d'un composé de formule (I) avec un composé de formule $R^7Y$, où $R^7$ est un groupe alkyle ayant jusqu'à 5 atomes de carbone et Y est un anion pharmaceutiquement acceptable.

**2.** Composé selon la revendication 1, dans lequel $R^2$ est un groupe phényle.

**3.** Composé selon la revendication 1, dans lequel $R^3$ est H.

**4.** Composé selon la revendication 1, de formule

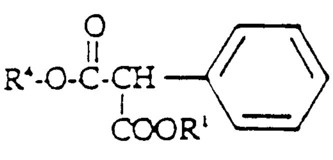

(Ia)

où $R^1$ est un groupe alkyle en chaîne droite ou ramifiée ou cycloalkyle ayant jusqu'à 8 atomes de carbone; et $R^4$ est

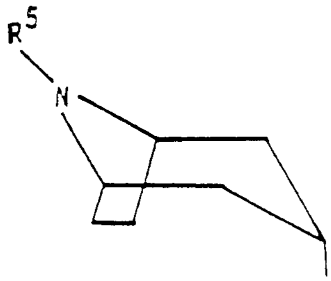

ou

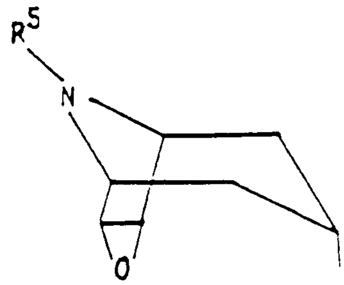

où $R^5$ est un groupe alkyle ayant jusou'à 5 atomes de carbone; ou un sel d'ammonium quaternaire d'un composé de formule (Ia) avec un composé de formule $R^7Y$, où $R^7$ est un groupe alkyle ayant juscu'à 5 atomes de carbone et Y est un anion pharmaceuticuement acceptable.

**5.** Composé selon l'une quelconque des revendications 1 à 4, dans lequel $R^1$ est un groupe cycloalkyle.

**6.** Composé selon l'une quelconque des revendications 1 à 4, dans lequel $R^1$ est un groupe alkyle en chaîne droite ou ramifiée.

**7.** Composé selon la revendication 6, dans lequel $R^1$ est un groupe méthyle, éthyle ou propyle.

**8.** Composé d'ammonium quaternaire selon l'une quelconque des revendications 1 à 7, dans lequel $R^7$ est un groupe méthyle et Y est un radical halogénure, sulfate, alkylsulfate ou alkylsulfonate.

**9.** Composé selon la revendication 1, de formule

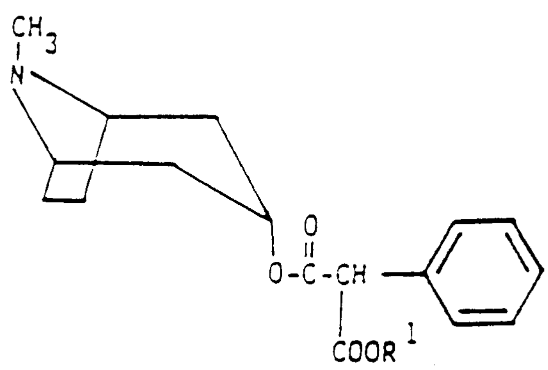

où $R^1$ est tel que défini dans la revendication 1.

**10.** Composé selon la revendication 9, dans lequel $R^1$ est un groupe cycloalkyle.

**11.** Composé selon la revendication 10, dans lequel $R^1$ est un groupe cyclohexyle.

**12.** Composé selon la revendication 9, dans lequel $R^9$ est un groupe alkyle en chaîne droite ou ramifiée.

**13.** Composé selon la revendication 12, dans lequel $R^1$ est un groupe méthyle.

**14.** Composé selon la revendication 12, dans lequel $R^1$ est un groupe éthyle.

**15.** Composé selon la revendication 12, dans lequel $R^1$ est un groupe isopropyle.

**16.** Composé selon l'une quelconque des revendications 9 à 15, sous la forme de son sel avec du diméthylsulfate.

**17.** Composé selon la revendication 1, de formule

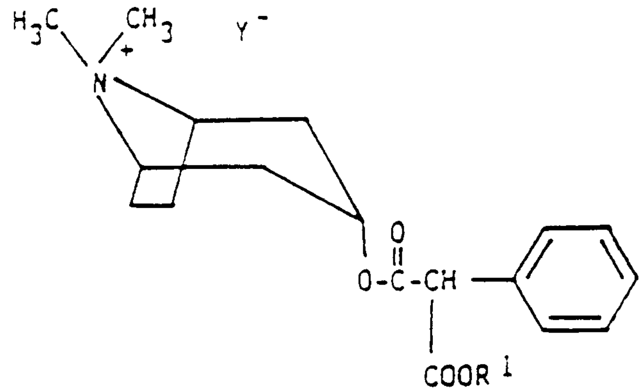

où $R^1$ et Y sont tels que définis dans la revendication 1.

**18.** Composé selon la revendication 17, dans lequel $R^1$ est un groupe cycloalkyle.

**19.** Composé selon la revendication 18, dans lequel $R^1$ est un groupe cyclohexyle.

**20.** Composé selon la revendication 17, dans lequel $R^1$ est un groupe alkyle en chaîne droite ou ramifiée.

**21.** Composé selon la revendication 20, dans lequel $R^1$ est un groupe méthyle.

**22.** Composé selon la revendication 20, dans lequel $R^1$ est un groupe éthyle.

**23.** Composé selon la revendication 20, dans lequel $R^1$ est un groupe isopropyle.

**24.** Composé selon l'une quelconque des revendications 17 à 23, dans lequel $Y^-$ est $I^-$ ou $CH_3SO_4^-$.

**25.** Composé selon la revendication 1, de formule

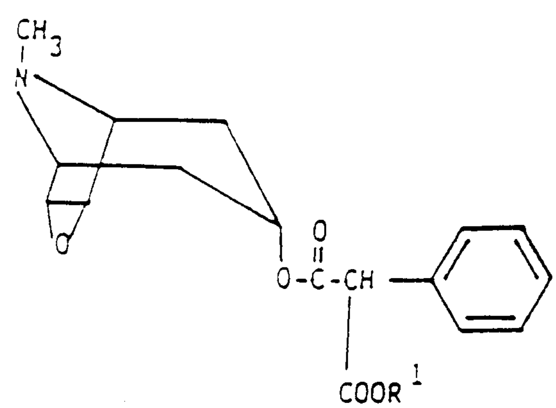

où $R^1$ est tel que défini dans la revendication 1.

**26.** Composé selon la revendication 1, de formule

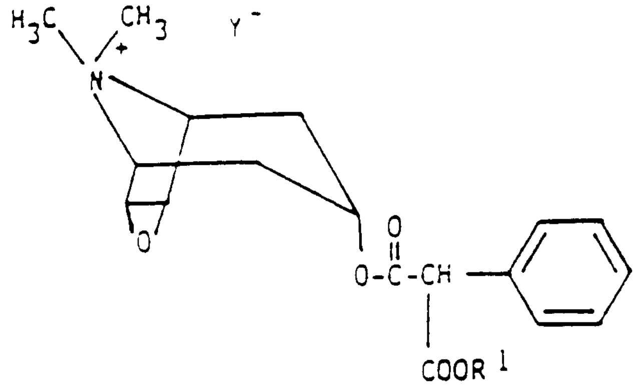

où $R^1$ et Y sont tels que définis dans la revendication 1.

**27.** Composition pharmaceutique sous forme d'unité de dosage comprenant une quantité anticholinergique efficace d'un composé selon l'une quelconque des revendications 1 à 26 et un véhicule ou support de celui-ci pharmaceutiquement acceptable.

**28.** Composition pharmaceutique sous forme d'unité de dosage pour une administration à l'oeil ou aux yeux d'un animal ou de l'homme, comprenant une quantité mydriatique efficace d'un composé selon l'une quelconque des revendications 1 à 26 et un véhicule ou support de celui-ci pharmaceutiquement acceptable.

**Revendications pour les Etats contractants suivants : AT, ES, GR**

**1.** Procédé pour la production d'un composé de formule

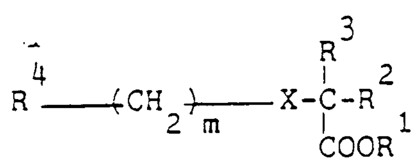

(I)

où

- $R^1$ est un groupe en chaîne alkyle droite ou ramifiée ou cycloalkyle ayant jusqu'à 8 atomes de carbone;
- $R^2$ est un groupe aryle, cycloalkyle ou alkyle ayant jusqu'à 8 atomes de carbone;
- $R^3$ est H ou un groupe défini par $R^2$;
- X est

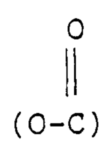

ou

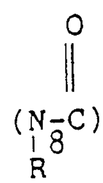

où $R^8$ est H ou un groupe alkyle en chaîne droite ou ramifiée ayant jusqu'à 5 atomes de carbone;

- m est un entier de 0 à 4; et
- $R^4$ est

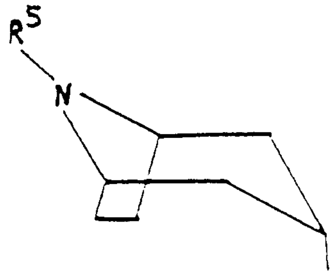

ou

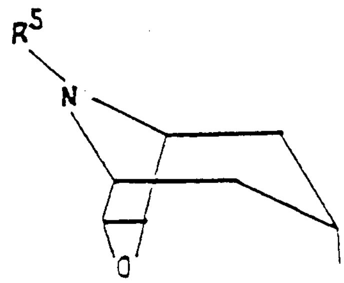

où $R^5$ est un groupe alkyle ayant jusqu'à 5 atomes de carbone; ou $R^4$ est

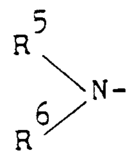

où $R^5$ et $R^6$ peuvent être identiques ou différents et sont des groupes alkyle ayant jusqu'à 5 atomes de carbone; ou $R^4$ est une alkyl pyridine ortho-, méta- ou para-substituée de formule

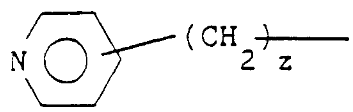

où Z est un entier de 0 à 3; ou un sel d'ammonium quaternaire d'un composé de formule (I) avec un composé de formule $R^7Y$, où $R^7$ est un groupe alkyle ayant jusqu'à 5 atomes de carbone et Y est un anion pharmaceutiquement acceptable,

les étapes comprenant la réaction d'un composé de formule II

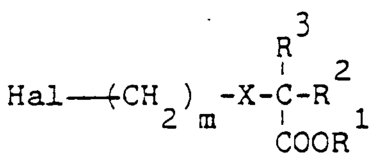

(II)

où

$R^1$, $R^2$, $R^3$, X et m ont la même signification que dans la formule I et Hal est un radical halogène, avec un composé de formule $R^4OH$, où $R^4$ a la même signification que dans la formule I, dans un solvant approprié et, éventuellement, la réaction du composé de formule I résultant avec un agent quaternaire approprié de formule $R^7Y$, où $R^7$ est un groupe alkyle ayant jusqu'à 5 atomes de carbone et Y est un anion pharmaceutiquement acceptable.

2. Procédé selon la revendication 1, dans lequel dans la formule II $R^2$ est un groupe phényle.

3. Procédé selon la revendication 1, dans lequel dans la formule II $R^3$ est H.

4. Procédé selon la revendication 1, dans lequel dans la formule II $R^1$ est un groupe alkyle en chaîne droite ou ramifiée ou cycloalkyle ayant jusqu'à 8 atomes de carbone; et dans lequel dans la formule $R^4OH$ $R^4$ est

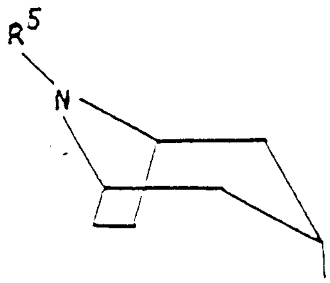

ou

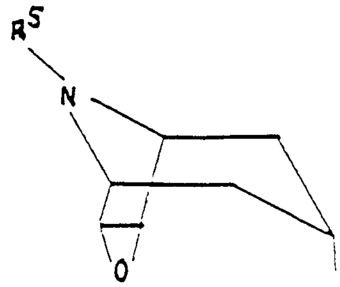

où $R^5$ est un groupe alkyle ayant jusqu'à 5 atomes de carbone.

**5.** Procédé selon l'une quelconque des revendications 1 à 4, dans lequel dans la formule II $R^1$ est un groupe cycloalkyle.

**6.** Procédé selon l'une quelconque des revendications 1 à 4, dans lequel dans la formule II $R^1$ est un groupe alkyle en chaîne droite ou ramifiée.

**7.** Procédé selon la revendication 6, dans lequel dans la formule II $R^1$ est un groupe méthyle, éthyle ou propyle.

**8.** Procédé selon l'une quelconque des revendications 1 à 7, dans lequel dans la formule $R^7Y$ $R^7$ est un groupe méthyle et Y est un radical halogénure, sulfate, alkylsulfate ou alkylsulfonate.

**9.** Procédé selon la revendication 1, dans lequel dans la formule II $R^2$ est un groupe phényle, $R^3$ est H, m est O et dans la formule $R^4OH$ $R^4$ est

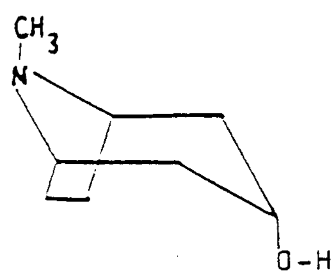

**10.** Procédé selon la revendication 9, dans lequel dans la formule II $R^1$ est un groupe cycloalkyle.

**11.** Procédé selon la revendication 10, dans lequel dans la formule II $R^1$ est un groupe cyclohexyle.

**12.** Procédé selon la revendication 9, dans lequel dans la formule II $R^1$ est un groupe alkyle en chaîne droite ou ramifiée.

**13.** Procédé selon la revendication 12, dans lequel dans la formule II $R^1$ est un groupe méthyle.

**14.** Procédé selon la revendication 12, dans lequel dans la formule II $R^1$ est un groupe éthyle.

**15.** Procédé selon la revendication 12, dans lequel dans la formule II $R^1$ est un groupe isopropyle.

**16.** Procédé selon l'une quelconque des revendications 9 à 15, comprenant la réaction du produit résultant de formule I avec du diméthylsulfate pour obtenir le sel d'ammonium quaternaire correspondant.

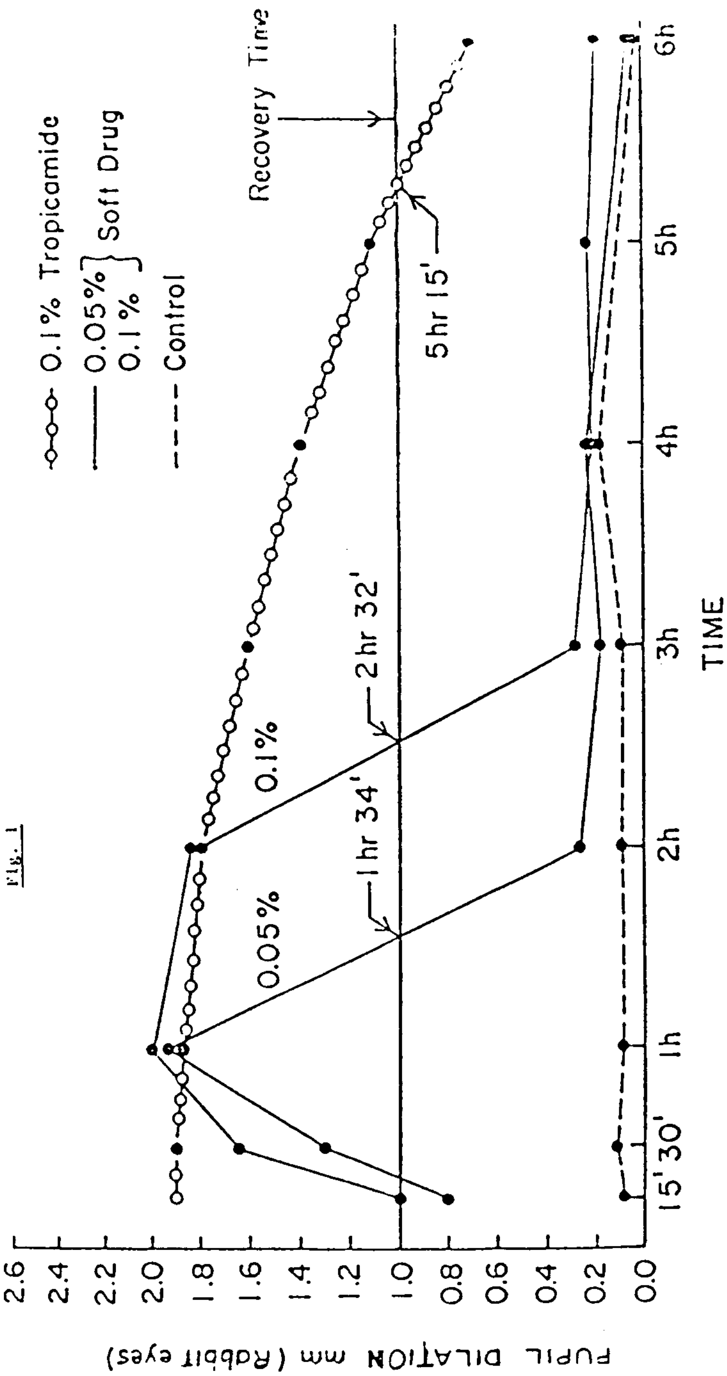
Fig. 1
0.1% Tropicamide
0.05%
0.1 %
Soft Drug
Control
Recovery Time
5hr 15'
2hr 32'
1hr 34'
0.1%
0.05%
PUPIL DILATION mm (Rabbit eyes)
2.6
2.4
2.2
2.0
1.8
1.6
1.4
1.2
1.0
0.8
0.6
0.4
0.2
0.0
15' 30'
1h
2h
3h
4h
5h
6h
TIME